# EUROPEAN PATENT APPLICATION

(11) **EP 2 959 820 A1**
(43) Date of publication of application: **30.12.2015**
(21) Application number: 14753899.5
(22) Date of filing: 18.02.2014
(51) Int. Cl.: A61B 1/00

(54) **SUBJECT INSERTION SYSTEM**

(30) Priority: 21.02.2013 JP 2013032311
(71) Applicant: Olympus Corporation, Shibuya-ku Tokyo 151-0072 (JP)
(72) Inventor: HANE, Jun, Tokyo 151-0072 (JP); FUJITA, Hiromasa, Tokyo 151-0072 (JP); TOJO, Ryo, Tokyo 151-0072 (JP); ITO, Takeshi, Tokyo 151-0072 (JP)
(74) Representative: Winter, Brandl, Fürniss, Hübner, Röss, Kaiser, Polte - Partnerschaft mbB
(86) International application number: PCT/JP2014/053705
(87) International publication number: WO 2014/129436

(57) **Abstract**

An object insertion system (10) includes an insertion portion (20), a first information generator (101), a storage unit (17), a second information calculator (102) and an output unit (16). The first information generator (101) generates first information indicating at least one of an insertion state and an operation state of the insertion portion (20) in a body cavity (lumen). The second information calculator (102) calculates second information based on the first information generated by the first information generator (101) and at least one of third information and fourth information stored by the storage unit (17), the second information being operation support information to insert the insertion portion 20 to perform a predetermined work. The output unit (16) outputs the second information.

## Description

### Technical Field

The present invention relates to an object insertion system which is inserted into an object to conduct various procedures such as observation, repair, treatment, and acquisition.

### Background Art

There has been known a device which is used so that an insertion portion thereof is inserted into a body cavity (lumen), as in an endoscope. In the case of such a device, it is not possible to directly ascertain the state (e.g. shape) of the insertion portion by surgeon's visual observation after the insertion portion has been inserted in an object, and the relation between an image acquired by the use of, for example, an endoscope and an actual observation place is unclear. Therefore, the surgeon may mistake the insertion direction and curving direction of the insertion portion.

Under these circumstances, there have been suggested various techniques which incorporate sensors for, for example, shape detection, position detection, and force detection in the insertion portion of the endoscope so that, for example, the insertion can be easily and safely operated, that is, so that the shape of the insertion portion of the endoscope inserted in the object can be recognized.

For example, Jpn. Pat. Appln. KOKAI Publication No. 2007-130175 (PTL 1) discloses an endoscope insertion portion shape recognition system for recognizing the shape of an insertion portion of a flexible endoscope. Specifically, PTL 1 discloses including position detection means for detecting the positions of both ends of a curving portion in the insertion portion and the position of at least one point in the curving portion, curving state detection means for detecting the curving state of each of positions of the curving portion, and curving portion shape reproduction means for reproducing the shape of the curving portion from positions of both the ends, the position of the point in the curving portion, and the curving state of the above portions.

### Citation List

### Patent Literature

PTL 1: Jpn. Pat. Appln. KOKAI Publication No. 2007-130175

### Summary of Invention

### Technical Problem

An operator of the endoscope such as a doctor has matters to consider and examine in addition to matters associated with the insertion portion of the endoscope. These matters include various matters such as the condition of a patient, an estimated position of the affected part, and diagnosis and treatment methods. An operator who is inexperienced in use of the endoscopic system has difficulty in understanding information regarding the insertion portion of the endoscope. Therefore, even when provided with sensor information regarding the insertion portion of the endoscope, the operator cannot make an instantaneous judgment on the basis of the sensor information and then take proper measures unless the sensor information is easy for operator to understand, facilitates the understanding of the importance, and is easy to utilize.

For example, although sensor information which is estimated to be generally useful is provided, reading this sensor information may be complicated or necessary information may be insufficient if this sensor information is inconsistent with the condition of the patient or is unnecessary information.

Accordingly, at present, there are demands for an object insertion system for providing, by proper timing, the operator with useful information to assist in the insertion of the endoscope and in various operations. It is preferable that this system can properly add and update information obtained at the time of the insertion of the endoscope and various operations.

The present invention has been made in view of the circumstances, and is intended to provide an object insertion system which can provide not only sensor information as information regarding an insertion portion but also useful information to assist in an operator's operation.

### Solution to Problem

An object insertion system according to an aspect of the invention includes an insertion portion which is inserted into a body cavity (lumen) of an object to perform predetermined work, a first information generator which generates first information, the first information being information indicating at least one of an insertion state and an operation state of the insertion portion in the body cavity (lumen), a storage unit which stores at least one of third information regarding the object and fourth information associated with the work, a second information calculator which calculates second information based on the first information and at least one of the third information and the fourth information, the second information being operation support information to insert the insertion portion to perform the predetermined work, and an output unit which outputs the second information.

### Advantageous Effects of Invention

According to the present invention, it is possible to provide an object insertion system which can provide not only sensor information as information regarding an insertion portion but also useful information to assist in an operator's operation.

### Brief Description of Drawings

FIG. 1 is a diagram showing one configuration example of an object insertion system according to a first embodiment of the present invention;
FIG. 2 is a schematic configuration diagram showing a curving operation amount detection/calculation mechanism;
FIG. 3 is a schematic configuration diagram showing a curving shape detection/calculation mechanism;
FIG. 4 is a diagram showing one configuration example of a fiber sensor;
FIG. 5 is a diagram showing one configuration example of a fiber sensor;
FIG. 6 is a diagram showing one configuration example of a fiber sensor;
FIG. 7 is a diagram showing an example of how an insertion portion sensor is disposed in the entrance (oral cavity) of a body cavity (lumen) of an object (patient);
FIG. 8 is a diagram showing the concept of the detection of the shape of a curving portion;
FIG. 9 is a block diagram showing the flow of information regarding support information presentation processing in the object insertion system according to the first embodiment of the present invention;
FIG. 10 is a diagram showing an endoscope inserted in the body cavity and parts associated with work;
FIG. 11 is a diagram showing the flow of the generation of second information;
FIG. 12 is a diagram showing the endoscope inserted in the body cavity and the situation of arrival at a workable range;
FIG. 13 is a diagram showing the flow of the generation of second information;
FIG. 14 is a diagram showing the endoscope inserted in the body cavity and the situation of arrival at a workable range;
FIG. 15 is a diagram showing the flow of the generation of second information;
FIG. 16 is a diagram showing a specific operation example of the endoscope;
FIG. 17 is a diagram showing the flow of the generation of second information;
FIG. 18 is a diagram showing the flow of the generation of second information;
FIG. 19 is a diagram showing the flow of the generation of second information;
FIG. 20 is a diagram showing the flow of the generation of second information;
FIG. 21 is a diagram showing the flow of the generation of second information;
FIG. 22 is a diagram showing a flowchart of a series of processing associated with the second information by a controller (second information calculator) in the object insertion system according to the first embodiment of the present invention;
FIG. 23 is a diagram showing one configuration example of an object insertion system according to a second embodiment of the present invention;
FIG. 24 is a diagram showing one configuration example of an object insertion system according to a third embodiment of the present invention;
FIG. 25 is a diagram showing the concept of switch control associated with the second information by the controller;
FIG. 26 is a diagram showing examples of setting information in which the second information, a combination of input information necessary for the generation of the second information, and utilization scenes of the second information are associated;
FIG. 27 is a diagram showing the concept of switch control associated with the second information by the controller;
FIG. 28 is a diagram showing an example of grade classification associated with the selection and switch of the second information;
FIG. 29 is a diagram showing the concept of switch control associated with the second information by the controller;
FIG. 30 is a diagram showing examples of setting information regarding output switch in which the second information, output means, and output methods are associated.

### Description of Embodiments

Embodiments of the present invention will be described below with reference to the drawings.

### [First Embodiment]

In a first embodiment, a medical endoscopic system to which an object insertion system 10 is applied is described by way of example. The object insertion system 10 is applicable to general purposes other than the medical endoscopic system as long as the object insertion system 10 operates an insertion portion 20, is inserted into an object, and treats the inside of the object. The object insertion system 10 is also applicable to, for example, a hard endoscope, a catheter, and a medical manipulator.

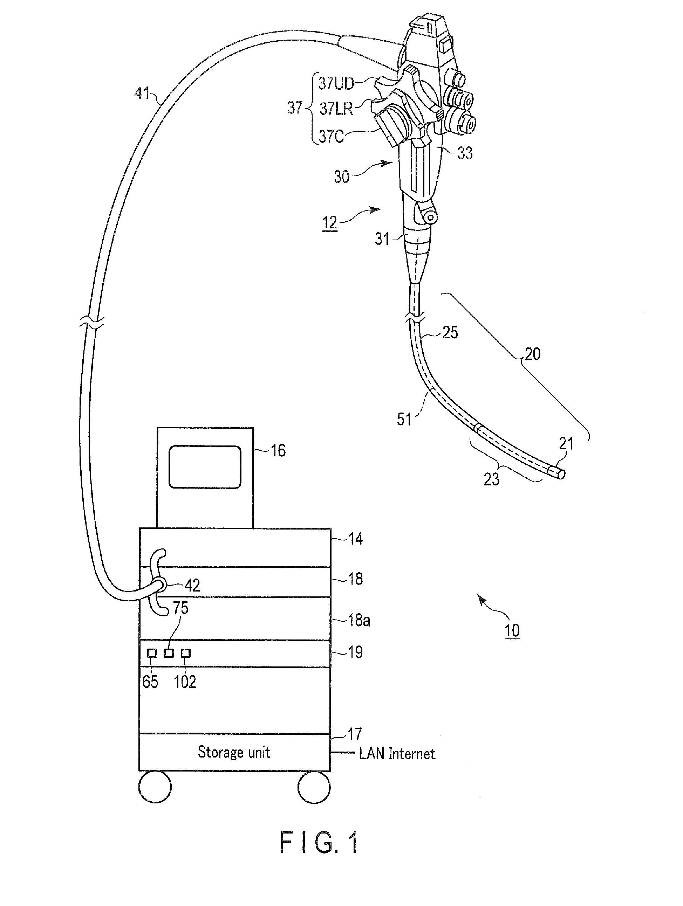

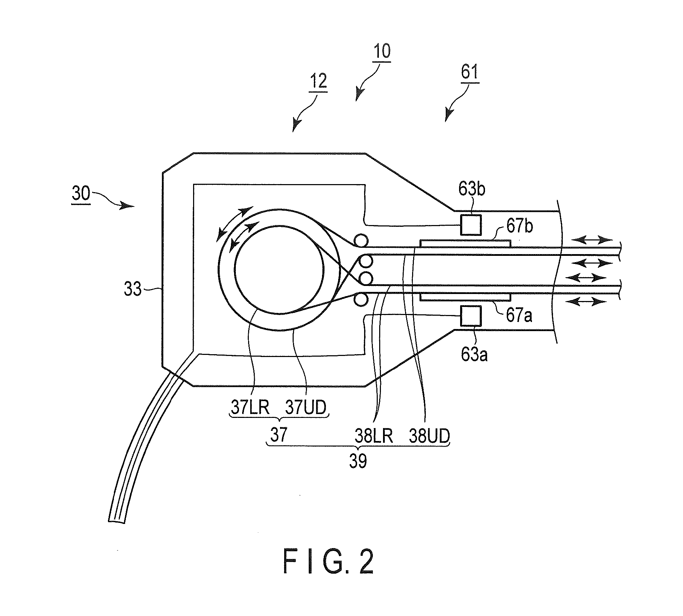

FIG. 1 is a diagram showing one configuration example of the object insertion system 10 according to the present embodiment. FIG. 2 is a schematic configuration diagram of a curving operation amount detection/calculation mechanism. The object insertion system 10 includes an endoscope 12, an image processor 14, an output unit 16, a storage unit 17, a light source device 18, a light emission detection unit 18a, and a controller 19. The image processor 14, the light source device 18, the light emission detection unit 18a, and the controller 19 are connected to one another. The image processor 14, the light source device 18, and the light emission detection unit 18a are attachable to and detachable from the endoscope 12 via a connector 42, respectively.

The endoscope 12 includes the tubular insertion portion 20. The endoscope 12 inserts the insertion portion 20 into a body cavity (lumen) of an object. This endoscope 12, for example, images a desired observation target such as an affected part or a lesion in the body cavity (lumen) of the object.

The image processor 14 subjects image data acquired by the imaging in the endoscope 12 to predetermined image processing. The image processor 14 is, for example, a video processor.

The output unit 16 outputs second information Q2 which is information for assisting in the operation of the endoscope 12 and presents the second information Q2 to an operator. The output unit 16 is display means such as a monitor, and displays, for example, the image data output from the image processor 14 on the monitor. The output unit 16 is not limited to the display means, and has only to output the second information Q2 by, for example, sound.

The storage unit 17 stores various information, for example, at least one of third information Q3 including information regarding the object and fourth information Q4 including information regarding work. The storage unit 17 stores the second information Q2 calculated by a second information calculator 102 in association with at least one of first information Q1, the third information Q3, and fifth information Q5.

The light source device 18 emits illumination light. The illumination light emitted from the light source device 18 is transmitted to the endoscope 12.

The light emission detection unit 18a emits light which is different in, for example, wavelength from the illumination light emitted from the light source device 18, and detects the emitted light.

The controller 19 conducts overall control of the whole object insertion system 10.

The endoscope 12 includes the insertion portion 20 and an operation portion 30.

The insertion portion 20 is in a hollow, diametrically small, and long shape, and is inserted into a body cavity (lumen) of a patient. The insertion portion 20 includes a distal hard portion 21, a curving portion 23, and a flexible tubular portion 25. The distal hard portion 21, the curving portion 23, and the flexible tubular portion 25 are provided from the distal side of the insertion portion 20 to the proximal side. The proximal end of the distal hard portion 21 is coupled to the distal end of the curving portion 23. The proximal end of the curving portion 23 is coupled to the proximal end of the flexible tubular portion 25.

The distal hard portion 21 is formed at the distal end of the insertion portion 20, that is, at the distal end of the endoscope 12. The distal hard portion 21 is hard.

The curving portion 23 is constructed by joint rings that are arranged along the longitudinal axis direction of the insertion portion 20 and are rotatably coupled to one another. The curving portion 23 curves in response to the operator's operation on a curving operation portion 37. The operator operates the curving operation portion 37 so that the observation target in the object will be caught in an observation field of the endoscope 12 and so that the illumination light will illuminate the observation target. The curving portion 23 curves in desired directions such as, upward, downward, leftward, and rightward directions in accordance with the operator's operation on the curving operation portion 37. As a result of this curving, the distal hard portion 21 of the insertion portion 20 changes its position and direction.

The flexible tubular portion 25 is formed as a tubular member, and extends from a body portion 31. The flexible tubular portion 25 has predetermined flexibility, and is bent by the application of external force.

As shown in FIG. 1 and FIG. 2, the operation portion 30 includes a grasp portion 33, a universal cord 41, and a curving operation amount detection/calculation mechanism 61.

The grasp portion 33 is coupled to the proximal end of the body portion 31. The grasp portion 33 is grasped by, for example, the hand of the operator who operates the endoscope 12. One end of the universal cord 41 is connected to the grasp portion 33.

Specifically, as shown in FIG. 1 and FIG. 2, the curving operation portion 37 is provided in the grasp portion 33. The curving operation portion 37 drives each of operation wires 38LR and 38UD for curving the curving portion 23. The curving operation portion 37 includes a left-right curving operation knob 37LR for leftward and rightward curving operation of the curving portion 23, an up-down curving operation knob 37UD for upward and downward curving operation of the curving portion 23, and a fixed knob 37C for fixing the position of the curved curving portion 23.

The left-right curving operation knob 37LR curves the curving portion 23 in a left-right direction via a leftward/rightward curving operation driving unit and the operation wire 38LR as shown in FIG. 2. The leftward/rightward curving operation driving unit which is driven by the operator's operation on the left-right curving operation knob 37LR is connected to the left-right curving operation knob 37LR. The leftward/rightward curving operation driving unit is provided in, for example, the grasp portion 33. The leftward/rightward curving operation driving unit is connected to the operation wire 38LR which is inserted through the operation portion 30, the flexible tubular portion 25, and the curving portion 23. The operation wire 38LR is connected to the distal end of the curving portion 23.

The up-down curving operation knob 37UD curves the curving portion 23 upward and downward via an upward/downward curving operation driving unit and the operation wire 38UD as shown in FIG. 2. The upward/downward curving operation driving unit which is driven by the operator's operation on the up-down curving operation knob 37UD is connected to the up-down curving operation knob 37UD. The upward/downward curving operation driving unit is provided in, for example, the grasp portion 33. The upward/downward curving operation driving unit is connected to the operation wire 38UD which is inserted through the operation portion 30, the flexible tubular portion 25, and the curving portion 23.

The operation wire 38UD and the operation wire 38LR are wires which move independently of each other. The operation wire 38UD and the operation wire 38LR are respectively connected to the distal end of the curving portion 23.

A curving operation mechanism 39 curves the curving portion 23. An operation amount (curving operation amount) for curving the curving portion 23 by the curving operation mechanism 39 is the amount for operating each of the operation wires 38LR and 38UD and the curving operation portion 37. The curving operation mechanism 39 includes the curving operation portion (the left-right curving operation knob 37LR, the up-down curving operation knob 37UD) 37, the leftward/rightward curving operation driving unit, the operation wire 38LR, the upward/downward curving operation driving unit, and the operation wire 38UD.

The universal cord 41 extends from the side surface of the grasp portion 33. The connector 42 which is attachable to and detachable from the image processor 14, the light source device 18, and the light emission detection unit 18a is provided at the other end of the universal cord 41.

The curving operation amount detection/calculation mechanism 61 includes two curving operation amount detection units (operation amount sensors) 63a and 63b which detect the curving operation amount of the curving operation mechanism 39 as shown in FIG. 2, a curving operation amount calculator 65 (FIG. 1) which calculates curving operation amount information indicating an operation amount on the basis of the detection results by each of the curving operation amount detection units 63a and 63b, and two read target portions 67a and 67b respectively facing the curving operation amount detection units 63a and 63b. The curving operation amount calculator 65 is provided in, for example, the controller 19.

Each of the two curving operation amount detection units 63a and 63b detects the curving operation amount by the curving operation mechanism 39 shown in FIG. 2. Each of the curving operation amount detection units 63a and 63b is, for example, a linear encoder, and is provided inside the operation portion 30.

One read target portion 67a is provided, for example, at the proximal end of the operation wire 38LR. The other read target portion 67b is provided, for example, at the proximal end of the operation wire 38UD. The read target portion 67a moves together with the movement of the operation wire 38LR. The read target portion 67b moves together with the movement of the operation wire 38UD. Each of the read target portions 67a and 67b is, for example, a linear scale.

One curving operation amount detection unit 63a is provided to face one read target portion 67a. The curving operation amount detection unit 63a reads the read target portion 67a which moves together with the operation wire 38LR to detect the moving position of the read target portion 67a, and detects the movement of the operation wire 38LR from the moving position of the read target portion 67a.

The other curving operation amount detection unit 63b is provided to face the other read target portion 67b. The curving operation amount detection unit 63b reads the read target portion 67b which moves together with the operation wire 38UD to detect the moving position of the read target portion 67b, and detects the movement of the operation wire 38UD from the moving position of the read target portion 67b.

The curving operation amount calculator 65 detects the movement amount of each of the read target portions 67 and 67b, that is, the movement amount of each of the operation wires 38LR and 38UD on the basis of the moving position of each of the read target portions 67a and 67b detected by each of the curving operation amount detection units 63a and 63b. The curving operation amount calculator 65 calculates the curving operation amount of the curving portion 23 by the curving operation mechanism 39, that is, the curving operation amount information regarding each of the operation wires 38LR and 38UD on the basis of the movement amount of each of the operation wires 38LR and 38UD.

One read target portion 67a may be provided in the left-right curving operation knob 37LR, and the other read target portion 67b may be provided in the up-down curving operation knob 37UD. In this case, one read target portion 67a is provided, for example, in the outer circumferential surface of the circular cylindrical left-right curving operation knob 37LR, and the other read target portion 67b is provided, for example, in the outer circumferential surface of the circular cylindrical up-down curving operation knob 37UD.

One read target portion 67a may be provided in the surface of the left-right curving operation knob 37LR, and the other read target portion 67b may be provided in the surface of the up-down curving operation knob 37UD.

The curving operation amount detection unit 63a reads the read target portion 67a which rotates together with the left-right curving operation knob 37LR, and detects the rotation position of the read target portion 67a.

The curving operation amount detection unit 63b reads the read target portion 67b which rotates together with the up-down curving operation knob 37UD, and detects the rotation position of the read target portion 67b.

The curving operation amount calculator 65 calculates and computes the movement amount of each of the read target portions 67a and 67b, that is, the rotation amount of the left-right curving operation knob 37LR and the rotation amount of the up-down curving operation knob 37UD on the basis of the rotation position of each of the read target portions 67a and 67b which is the detection result by each of the curving operation amount detection units 63a and 63b.

The curving operation amount calculator 65 calculates and computes the curving operation amount information regarding each of the left-right curving operation knob 37LR and the up-down curving operation knob 37UD on the basis of the rotation amount of each of the left-right curving operation knob 37LR and the up-down curving operation knob 37UD.

The curving operation amount calculator 65 calculates the curving operation amount information regarding each of the left-right curving operation knob 37LR and the up-down curving operation knob 37UD, and thereby calculates the curving operation amount of the curving operation mechanism 39 to acquire its curving operation amount information.

Therefore, the curving operation amount detection/calculation mechanism 61 calculates the curving operation amount of the curving operation mechanism 39 to compute its curving operation amount information on the basis of the rotation amount of each of the left-right curving operation knob 37LR and the up-down curving operation knob 37UD in the curving operation mechanism 39.

The curving portion 23 does not always need to be able to curve upward, downward, leftward, and rightward, and may be able to only curve upward and downward or only curve leftward and rightward. In this case, the curving operation amount detection/calculation mechanism 61 detects the upward and downward curving operation amount or leftward and rightward curving operation amount of the curving operation mechanism 39, and calculates the respective curving operation amount information.

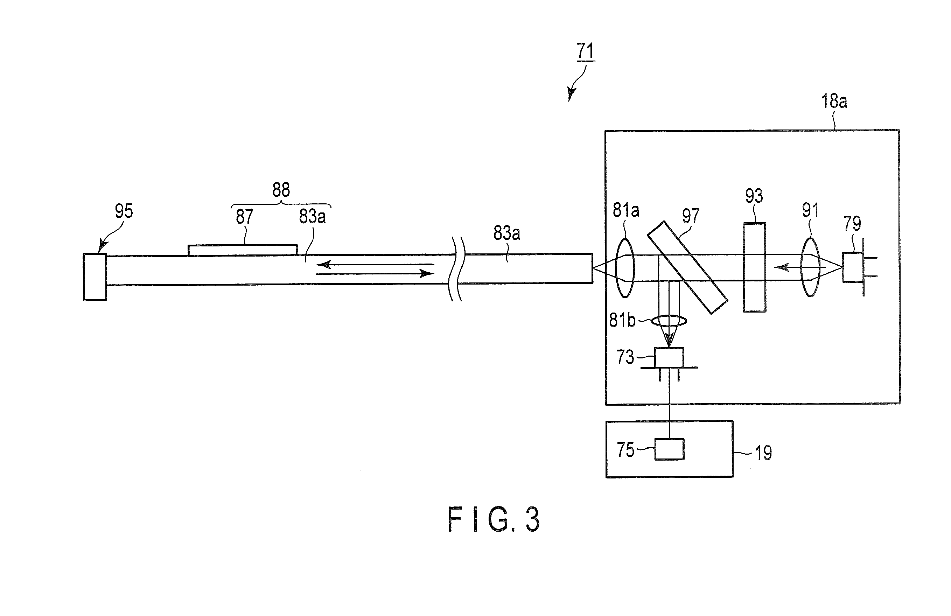

FIG. 3 shows a schematic configuration diagram of a curving shape detection/calculation mechanism 71. The curving shape detection/calculation mechanism 71 includes the light emission detection unit 18a, the controller 19, an optical fiber 83a, a detection region (light detection portion) 87, and a reflecting portion 95. The curving shape detection/calculation mechanism 71 detects the curving shape (curving amount) of the curving portion 23 which is actually curving, and calculates and computes curving shape information indicating the curving shape.

The light emission detection unit 18a includes a light source 79, a projection lens 91, an isolator 93, a reflecting mirror 97, a collecting lens 81a, a collecting lens 81b, and a curving shape detector 73.

The light source 79 is, for example, an LED, and emits light. The projection lens 91, the isolator 93, the reflecting mirror 97, and the collecting lens 81a are arranged on the optical path of the light emitted from the light source 79. The collecting lens 81b and the curving shape detector 73 are arranged on the reflection optical path of the reflecting mirror 97.

The projection lens 91 projects the light emitted from the light source 79.

The isolator 93 transmits light from one direction, and blocks light from other directions. The isolator 93 transmits the light emitted from the light source 79, and blocks light from the opposite direction. As a result, the light which has been transmitted by the isolator 93 is collected by the collecting lens 81a and then enters the optical fiber 83a.

The collecting lens 81a is provided between the light source 79 and the optical fiber 83a. The collecting lens 81a collects the light emitted from the light source 79 into the optical fiber 83a so that this light enters the optical fiber 83a.

The collecting lens 81b collects, into the curving shape detector 73, the light which has reflected back to the optical fiber 83a by the distal hard portion 21 provided at the distal end of the optical fiber 83a, passed through the collecting lens 81a, and reflected by the reflecting mirror 97.

The reflecting portion 95 reflects the light emitted from the optical fiber 83a, and allows the light to again enter the optical fiber 83a.

The reflecting mirror 97 transmits light from one direction, and reflects light from other directions. That is, the reflecting mirror 97 transmits, toward the collecting lens 81a, the light which has been emitted from the light source 79 and which has passed through the projection lens 91 and the isolator 93, and reflects the light which has been emitted from the optical fiber 83a and which has passed through the collecting lens 81a.

The curving shape detector 73 includes, for example, a light receiving portion such as a light receiving element. The curving shape detector 73 receives incident light, and outputs a light receiving signal corresponding to, for example, the intensity of the received light. The curving shape detector 73 outputs a light receiving signal corresponding to the curving shape of the curving portion 23, for example, a curving direction and a curving degree (curving amount), on the basis of the light receiving signal.

The controller 19 conducts overall control of each unit of the object insertion system 10, and includes a curving shape calculator 75 as shown in FIG. 3. The curving shape calculator 75 calculates and computes the curving amount, curving direction, and curving shape of the curving portion 23 which is actually curving, on the basis of the light receiving signal output from the curving shape detector 73. The curving amount, curving direction, and curving shape of the curving portion 23 is computed on the basis of the change of the optical characteristics of the light guided into the optical fiber 83a, for example, the change of the light amount, by the detection region 87 provided in the optical fiber 83a.

The optical fiber 83a guides the light which has been emitted from the light source 79 and which has been collected by the collecting lens 81 to the distal hard portion 21 of the insertion portion 20 via the operation portion 30, as shown in FIG. 1. The optical fiber 83a is formed by a linear member.

At least one detection region 87 is provided in the optical fiber 83a. The detection region 87 forms a fiber sensor 88 as a shape sensor which detects the curving shape as shown in FIG. 3. The fiber sensor 88 is also referred to as a bend sensor. The fiber sensor 88 is a sensor of a light amount change detection type.

The detection region 87 emits the light to be guided into the optical fiber 83a to the outside of the optical fiber 83a in accordance with the curving state of the optical fiber 83a when the curving portion 23 of the optical fiber 83a curves. The light amount emitted to the outside of the optical fiber 83a corresponds to the curving amount of the optical fiber 83a. The detection region 87 is processed to leak the amount of light corresponding to the curving amount of the optical fiber 83a to the outside of the optical fiber 83a. In other words, the detection region 87 changes the optical characteristics, for example, the amount of light guided by the optical fiber 83a in accordance with the curving state of the insertion portion 20 (the detection region is optical characteristic changing unit). The detection region 87 is provided at least at or in the vicinity of the place where the curving of the insertion portion 20 is to be detected, for example, provided in the curving portion 23.

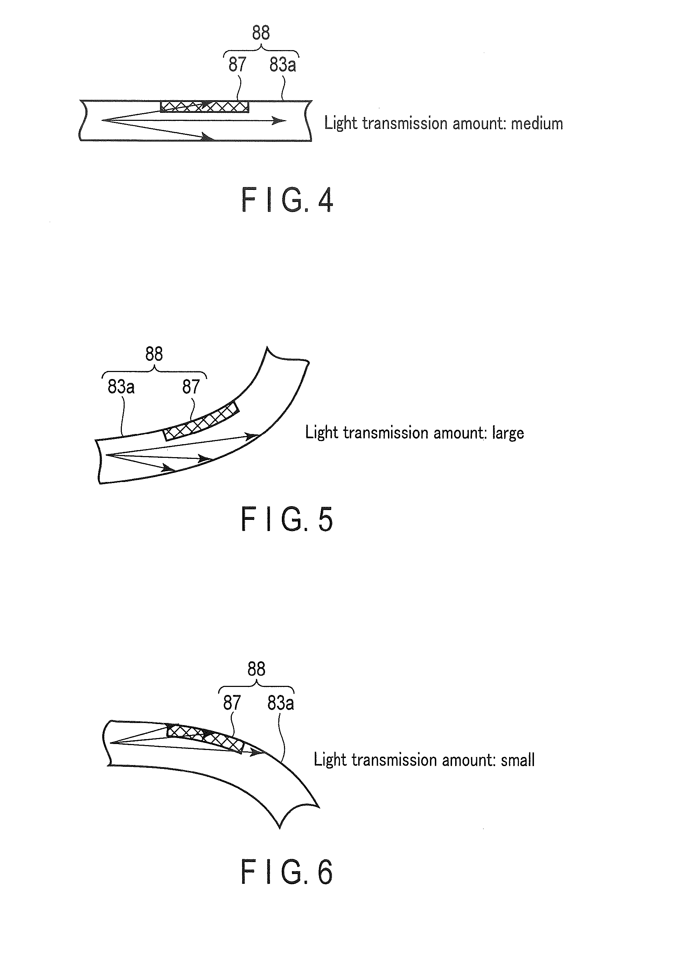

FIG. 4 to FIG. 6 show one configuration example of the fiber sensor 88. The fiber sensor 88 detects the curving shape (curving amount) of the insertion portion 20 including the curving portion 23. The fiber sensor 88 has the optical fiber 83a provided along the curving portion 23 of the insertion portion 20, and the detection region 87 provided at a particular place in the curving portion 23. The fiber sensor 88 is intended to compute the curving amount from the curvature of the optical fiber 83a.

If the optical fiber 83a changes from an uncurving first state (straight state) shown in FIG. 4 to a curving state shown in, for example, FIG. 5 or FIG. 6, the amount of light to enter the detection region 87 provided in the optical fiber 83a changes. FIG. 5 shows a second state in which the optical fiber 83a has curved so that the side where the detection region 87 is provided is the inner side of curving. FIG. 6 shows a third state in which the optical fiber 83a has curved so that the side where the detection region 87 is provided is the outer side of curving. If the first to third states are compared, the transmission amount of light by the optical fiber 83a is the greatest in the second state shown in FIG. 5, and the transmission amount of light by the optical fiber 83a is the smallest in the third state shown in FIG. 6.

If the fiber sensor 88 is used, the curving shape calculator 75 calculates the curving amount, curving direction, and curving shape of the curving portion 23 which is actually curving, and the curvature of the optical fiber 83a, on the basis of the light receiving signal output from the curving shape detector 73, that is, the change of the amount of light that enters the detection region 87.

The fiber sensor 88 is the sensor of the light amount change detection type as described above, and outputs the light receiving signal corresponding to the light amount that changes in accordance with the curving of the curving portion 23 (the bending of the optical fiber 83a), that is, the light amount which passes through the optical fiber 83a. Therefore, the fiber sensor 88 enables a sensor for detecting the curving shape (curving amount) of the optical fiber 83a to be inexpensively configured, and is suited to mass production.

The fiber sensor 88 is not limited to the light amount change detection type, and it is also possible to use a type in which grating is formed in an optical fiber called an FBG type. In the fiber sensor 88 of this type, the above-mentioned detecting portion is complicated and expensive, but multiple detection points can be provided in one optical fiber, or bending can be accurately detected.

More than one fiber sensor 88 may be used as sensors for detecting the curving shape. These sensors enable the detection of the curving shape in a desired range of the insertion portion 20, in particular, the curving portion 23 of the endoscope 12. Moreover, these sensors are small in diameter and can be easily incorporated in the curving portion 23, and can be unsusceptible to other components.

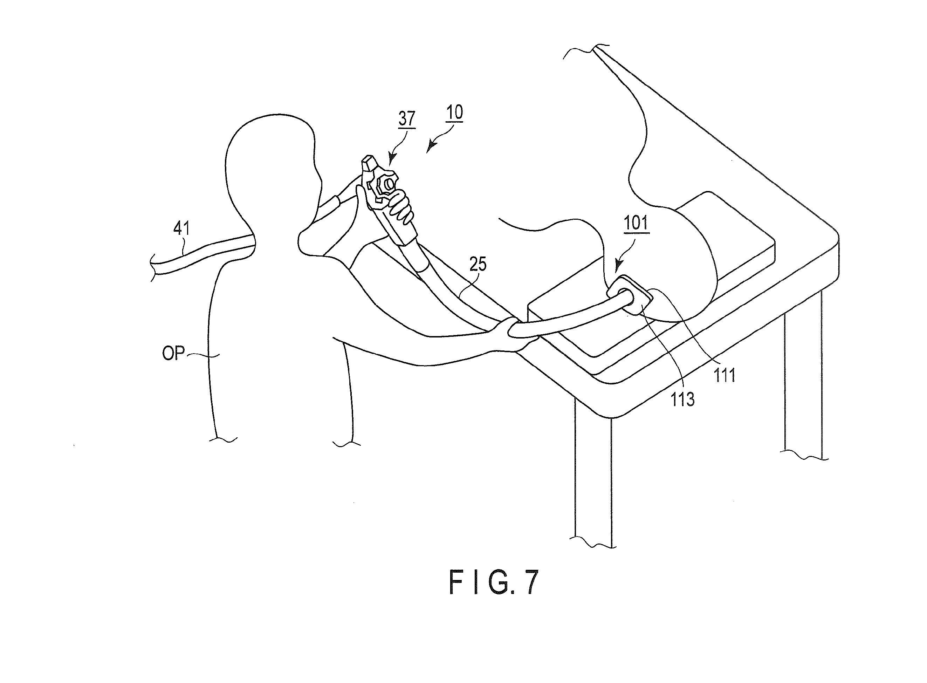

FIG. 7 shows an example of how an insertion portion sensor 113 is disposed in the entrance (an oral cavity 111) of a lumen of the object (patient). In the first embodiment, the insertion portion 20 of the endoscope 12 is inserted into (the gastrointestinal tract in) the body cavity (lumen) of the object (patient) after the insertion portion sensor 113 is disposed in the mouth 111 by the operation of an operator OP. The insertion portion sensor 113 detects the insertion amount and turning amount of the insertion portion 20 relative to the body cavity (lumen).

In the first embodiment, each of the curving operation amount detection units 63a and 63b which is the operation amount sensor, the fiber sensor 88 which is the shape sensor, and the insertion portion sensor 113 are provided as sensors.

The curving shape calculator 75 computes the insertion length and turning amount of the insertion portion 20 and the curving shape of the insertion portion 20 from the entrance of the body cavity (lumen) on the basis of the curving shape (curving amount) of the insertion portion 20 detected by the fiber sensor 88 and the insertion amount and turning amount of the insertion portion 20 into the body cavity (lumen) detected by the insertion portion sensor 113. As a result, the curving shape calculator 75 computes the shape, arrangement, distal position, and direction of the insertion portion 20 in the body cavity (lumen) on the basis of the insertion length and turning amount of the insertion portion 20 and the curving shape of the insertion portion 20.

The curving shape calculator 75 estimates the shape of the curving portion 23 on the basis of the curving operation amount of the curving operation mechanism 39 detected by each of the curving operation amount detection units 63a and 63b and the curving shape (curving amount) of the insertion portion 20 detected by the fiber sensor 88.

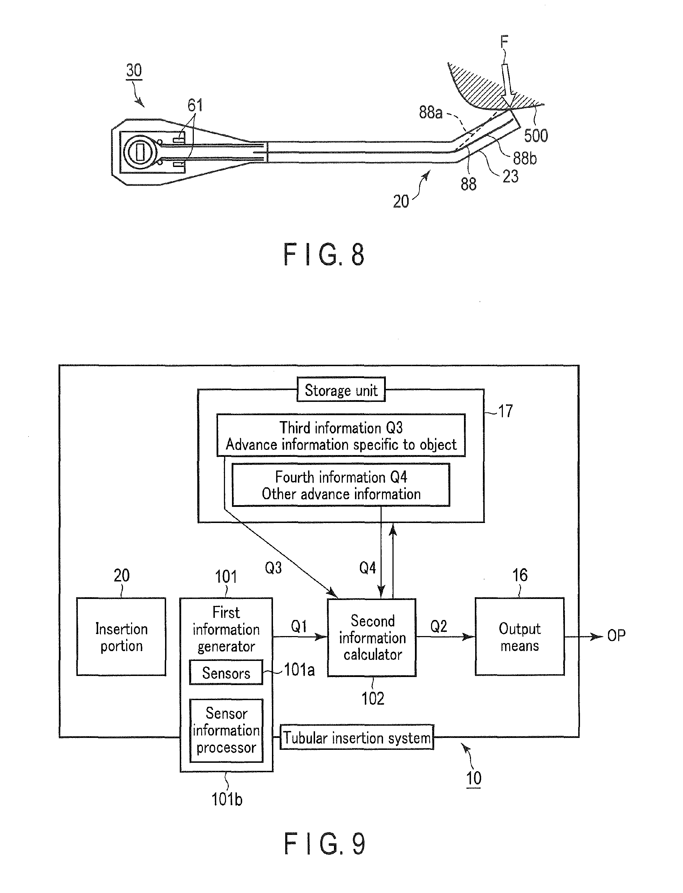

FIG. 8 shows the concept of the detection of the shape of the curving portion 23 in the insertion portion 20. If the distal part of the curving portion 23 collides with a lining 500 of the body cavity (lumen), force F is applied to the curving portion 23. In this case, the difference between the curving operation amount of the curving operation mechanism 39 detected by the curving operation amount detection unit 63 and the curving shape (curving amount) of the insertion portion 20 detected by the fiber sensor 88 is a value corresponding to the force F applied to the curving portion 23.

The controller 19 of the object insertion system 10 includes a first information generator 101, the second information calculator 102, and the storage unit 17, as shown in FIG. 9. The second information calculator 102 calculates the force F on the basis of the difference between the curving operation amount (a dotted line 88a indicating the fiber sensor 88) of the curving operation mechanism 39 detected by the curving operation amount detection unit 63 and the curving shape (curving amount: a full line 88b of the fiber sensor 88) of the curving portion 23 detected by the fiber sensor 88.

The second information calculator 102 calculates the difference between the actual curving amount of the curving portion 23 which is the detection result by the fiber sensor 88 and the curving operation amount detected by the curving operation amount detection unit 63 as the operation amount sensor, and on the basis of this difference, calculates the force F applied to the insertion portion 20 by the lining 500 of the body cavity (lumen).

The detection method of the force F is one example. The detection method is not limited to the curving operation amount detection unit 63, the fiber sensor 88, and the insertion portion sensor 113 depending on the force to be detected, and the combination of these sensors is not limited either. The detection method is not limited to the above-described method that uses the curving operation amount detection unit 63, the fiber sensor 88, and the insertion portion sensor 113 either.

The curving operation amount of the curving operation mechanism 39 detected by each of the curving operation amount detection units 63a and 63b, the curving shape (curving amount) of the insertion portion 20 detected by the fiber sensor 88, and the insertion amount and turning amount of the insertion portion 20 detected by the insertion portion sensor 113 are hereinafter referred to as a sensor value SV.

Information acquired by processing the sensor value SV is referred to as the first information Q1. The first information Q1 includes information indicating the shape of the insertion portion 20, or information indicating the relative positions of the insertion portion 20 and the object.

In detail, the information indicating the relative positions of the insertion portion 20 and the object includes information indicating at least one of the insertion amount and turning amount of the insertion portion 20 relative to the object, or information indicating the contact state between the insertion portion 20 and the object.

Specifically, the first information Q1 includes, for example, the following:
(Example 1) A "first curving shape" indicating the shape of the insertion portion 20, and an "insertion portion insertion amount" indicating the insertion amount of the insertion portion 20 into (the gastrointestinal tract in) the body cavity (lumen) of the object (patient),
(Example 2) An "insertion portion turning amount" indicating the turning (rotation) amount of the insertion portion 20,
(Example 3) A "curving operation amount" indicating an operation amount associated with curving of the curving portion 23 of the insertion portion 20,
(Example 4) A "second curving shape" indicating the curving shape of the insertion portion 20 relative to the lumen-shaped body cavity, and
(Example 5) An "insertion portion applied force" indicating the force F applied to the distal end of the insertion portion 20.

The "insertion portion insertion amount", the "insertion portion turning amount", and the "curving operation amount" are information directly obtained from the insertion amount and turning amount of the insertion portion 20 detected by the insertion portion sensor 113, and the curving operation amount of the curving operation mechanism 39 detected by each of the curving operation amount detection units 63a and 63b.

In contrast, the "first curving shape", the "second curving shape", and the "insertion portion applied force" are information obtained by processing the "insertion portion insertion amount", the "insertion portion turning amount", and the "curving operation amount".

The "first curving shape" is calculated as the curving shape in a predetermined range of the insertion portion 20 on the basis of the curving shape (curving amount) of the insertion portion 20 detected by the fiber sensor 88.

The "second curving shape" is calculated on the basis of the "first curving shape", the "insertion portion insertion amount", and the "insertion portion turning amount".

The "insertion portion applied force" is calculated on the basis of the "first curving shape" and the "curving operation amount".

Multiple sensors for obtaining the above-mentioned first information Q1 may be provided. The sensors for obtaining the above-mentioned first information Q1 do not always need to be incorporated in the insertion portion 20.

FIG. 9 is a block diagram showing the flow of information regarding support information presentation processing in the object insertion system 10 according to the first embodiment. In this diagram, various kinds of instruction information such as information requests and flows of various kinds of initial setting data are omitted.

The object insertion system 10 includes the first information generator 101, the second information calculator 102, and the storage unit 17.

The first information generator 101 calculates the first information Q1, and includes each sensor 101a and a sensor information processor 101b. Each sensor 101a includes a sensor associated with the acquisition of the first information Q1. In the first embodiment, each sensor 101a includes the curving operation amount detection unit 63, the fiber sensor 88, and the insertion portion sensor 13. The sensor information processor 101b processes the sensor value SV acquired by the sensors 101a to calculate the first information Q1.

In other words, the first information generator 101 generates the first information Q1 indicating at least one of an insertion state of the insertion portion 20 into the body cavity (lumen) and an operation state of the insertion portion 20. The first information generator 101 includes the fiber sensor 88 provided in the optical fiber 83a. The fiber sensor 88 detects the curving amount of one or more parts in the insertion portion 20.

The sensors 101a included in the first information generator 101 and the sensor information processor 101b for processing the information collected by the sensors 101a may be combined or separately provided.

The second information calculator 102 calculates the second information Q2 on the basis of the first information Q1 calculated by the first information generator 101 and at least one of the third information Q3 and the fourth information Q4 stored in the storage unit 17. The second information Q2 is operation support information of the object insertion system 10.

Next, the support information presentation processing by the object insertion system 10 is described.

The insertion portion sensor 113 is disposed in the entrance (the oral cavity 111) of the lumen of the object (patient), as shown in FIG. 7. In this state, the insertion portion 20 of the endoscope 12 is inserted into (the gastrointestinal tract in) the body cavity (lumen) of the object (patient), and the insertion portion 20 senses (the gastrointestinal tract in) the body cavity (lumen) of the object (patient).

Each sensor 101a of the first information generator 101 includes the insertion portion sensor 113, the fiber sensor 88, and the curving operation amount detection unit 63. The insertion portion sensor 113 detects the insertion amount and turning amount of the insertion portion 20 relative to the body cavity (lumen). The fiber sensor 88 detects the curving shape (curving amount) of the insertion portion 20. Each of the curving operation amount detection units 63a and 63b detects the curving operation amount by the curving operation mechanism 39.

The sensor information processor 101b of the first information generator 101 calculates the first information Q1 produced by processing the sensor value SV acquired by the each sensor (the insertion portion sensor 113, the fiber sensor 88, and the curving operation amount detection unit 63) 101a. The first information generator 101 outputs the first information Q1 to the second information calculator 102.

When the entire first information generator 101 is provided on the side of the insertion portion 20, the first information generator 101 sends the first information Q1 to the second information calculator 102 after processing the information regarding the insertion portion 20, that is, the sensor value SV. As a result, the first information Q1 regarding the insertion portion 20 can be directly acquired. Moreover, no external component for, for example, a device connected from the outside of the object insertion system 10 is needed, and the load on the side of the controller 19 can be light.

The advantage of providing the sensors 101a of the first information generator 101 outside the insertion portion 20 is that the size of the insertion portion 20 can be reduced. The sensor information processor 101b of the first information generator 101 is collectively configured in the same processing system as the second information calculator 102, so that high-speed and high-volume information processing can be achieved by one component, and the sensor information processor 101b can be reduced in size.

The second information calculator 102 generates the second information Q2 which is information for assisting in the operation of the endoscope 12 by the operator OP on the basis of the first information Q1 output from the first information generator 101, and the third information Q3 and the fourth information Q4 output from the storage unit 17. The second information calculator 102 displays and outputs this second information Q2 on the output unit 16, for example, the monitor.

The second information Q2 includes, for example, an instruction to insert the insertion portion 20 into the object, or information indicating an operation instruction associated with the work using the insertion portion 20. The second information Q2 includes information indicating an operation instruction associated with the rotation and/or insertion and removal of the insertion portion 20 necessary to locate the distal part of the insertion portion 20 at a particular position in the object. The second information Q2 includes information indicating an operation instruction associated with the curving of the insertion portion 20 necessary to locate the distal part of the insertion portion 20 at a particular position in the object.

Input information necessary for the contents of the second information Q2, and the calculation contents for generating the second information Q2 are predetermined. The first information Q1 is always used in the generation of the second information Q2. In contrast, at least one of the third information Q3 and the fourth information Q4 has only to be used in the generation of the second information Q2.

When there is missing information in the generation of the second information Q2, the second information calculator 102 may read the missing information from the storage unit 17, input the missing information in response to the operation of the operator OP, or input the missing information from some other external device (e.g. a database).

The second information calculator 102 does not exclusively generate one piece of second information Q2, and may sequentially or simultaneously generate pieces of second information Q2 and simultaneously output the pieces of second information Q2.

The output unit 16 to provide the second information Q2 to the operator OP includes various means such as sound, vibration, or electrical stimulation instead of the monitor which displays images. A voice guide or warning sound by, for example, a speaker may be used in addition to characters, graphics, and pictures to present the second information Q2 to the operator OP. When the operator OP is devoted to work and unaware of the second information Q2 or it takes time to notice the second information Q2, a vibration device or an electrical stimulation device, for example, may be disposed on the operation portion 30 of the endoscope 12 grasped by the operator OP or on the limbs of the operator OP, and the body of the operator OP may be directly stimulated. The device which directly stimulates the operator OP may be used together with the other output unit 16.

The second information Q2 is not exclusively output from the output unit 16, and may be saved in the storage unit 17 or stored in an external device such as a database. Alternatively, the second information Q2 may be output from the output unit 16 and also saved in the storage unit 17, or stored in an external device such as a database. Thus, the second information Q2 can be read from the storage unit 17 or the external device and then again displayed on the output unit 16. Consequently, the operator OP more easily ascertains the second information Q2.

The second information Q2 includes, for example, the following:
(Example 1) "Information indicating specific operation contents", for example, the insertion and rotation of the insertion portion 20 and the curving operation of the curving portion 23,
(Example 2) "Operation related situation information" indicating the direction of the insertion of the insertion portion 20, and indicating whether the position where an operation such as observation or a treatment at a desired insertion place G such as an affected part (lesion) is possible is reached,
(Example 3) An "operation and work guideline information" regarding, for example, an instruction on the work (e.g. observation or treatment) to be done next, and
(Example 4) A "workable range arrival situation" which is relative position information regarding the distal part of the insertion portion 20 to a workable range which permits the distal part of the insertion portion 20 to do the work.

The third information Q3 and the fourth information Q4 are described below.

The third information Q3 and the fourth information Q4 are stored in the storage unit 17.

The third information Q3 is information specific to the object. The third information Q3 includes, for example, information indicating the medical history of the object, and information indicating past diagnostic results of the object. Specifically, the third information Q3 includes information indicating, for example, "past diagnostic record of the patient", "observation, diagnosis, and treatment information", "the position of the affected part (lesion)", "disease name/symptom name", "the size of the affected part", "the level/extent of the symptom", "the race of the patient", and "body shape, constitution, and medical checkup results". The information regarding the position of the affected part (lesion) may be an X-ray image or a CT image in which the position can be ascertained.

The fourth information Q4 includes various advance information that does not correspond to the third information Q3. The fourth information Q4 includes information indicating the policy of the work, information indicating the procedure of the work, information indicating the specification of an instrument associated with the work, information indicating the performance of the instrument associated with work, or advance information regarding the work.

Specifically, the fourth information Q4 includes information indicating, for example, "information regarding the policy and procedure of work such as a diagnosis or a treatment", "information indicating, for example, the specification of an endoscopic system", "an insertion route to the affected part (lesion)", "an observation/treatment target", "information to be acquired", "matters to be diagnosed", "treatment contents/procedure", and "points to notice in the insertion and the observation/treatment (e.g. fragile parts, and allowed times associated with the insertion/observation/treatment".

Next, a contents example of the second information in a series of operations using the endoscope 12 is specifically described.

Here, a series of operations intended to insert the endoscope 12 into the body cavity (lumen) to observe and treat the patient or the affected part are described by way of example.

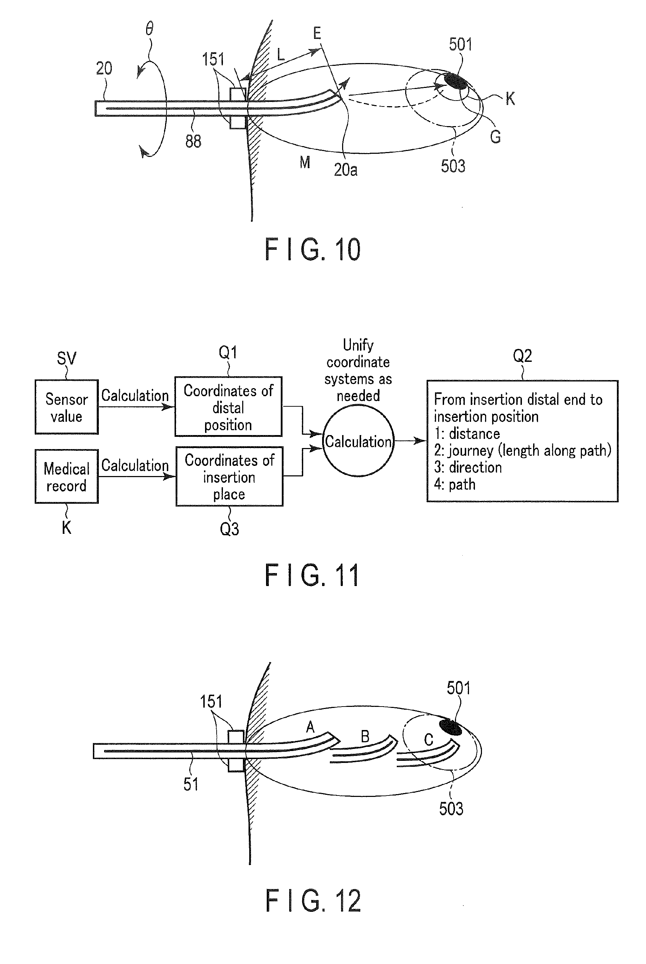

FIG. 10 shows the endoscope 12 inserted in a body cavity (lumen) M and parts associated with the work. FIG. 11 shows the flow of the generation of the second information Q2. K indicates a treatable range.

The first information Q1 includes information indicating a distal position 20a of the insertion portion 20 in the body cavity (lumen) M.

The third information Q3 includes work target position information which is the position information regarding an affected part or lesion 501.

The fourth information Q4 includes information indicating, for example, the insertion and observation regarding the affected part or lesion 501, and the contents and procedure of the treatment.

The second information calculator 102 generates the second information Q2 which is the relative position information regarding the distal position 20a of the insertion portion 20 and a certain work target position of the affected part, or intermediate information that can be processed into the second information Q2 on the basis of the first information Q1, the third information Q3, and the fourth information Q4.

When the endoscope 12 is inserted, the second information calculator 102 generates the second information Q2 on the basis of the first information Q1 and the third information Q3, as shown in FIG. 10 and FIG. 11. The second information Q2 includes, for example, the distance, journey (length along the path), direction, and path from the distal position 20a of the insertion portion 20 to the work target position.

Specifically, as shown in FIG. 11, the first information generator 101 sets a first coordinate system in the body cavity (lumen) M, performs a calculation for the sensor value SV to compute coordinates of the distal position 20a of the insertion portion 20 in the first coordinate system, and calculates the coordinates of the distal position 20a as the first information Q1.

The third information Q3 is generated as information including the work target position which is the position information regarding the affected part or lesion 501 from information K such as a medical record. The work target position of the affected part or lesion 501 indicates the coordinates of the place G where the distal end of the insertion portion 20 is inserted. A second coordinate system on, for example, the CT image including the affected part or lesion 501 is set, and the third information Q3 is generated as the work target position that is the position information regarding the affected part or lesion 501 on the second coordinate system. The third information Q3 is prestored in the storage unit 17.

The second information calculator 102 then calculates the second information Q2 on the basis of the coordinates of the distal position 20a of the insertion portion 20 which is the first information Q1, and the coordinates of the work target position which is the third information Q3. If the first coordinate system of the first information Q1 does not correspond to the second coordinate system of the third information Q3, the second information calculator 102 transforms one or both of the first and second coordinate systems, and represents the first information Q1 and the third information Q3 on the same coordinate system.

Next, one example of a calculating method of the second information Q2 regarding the insertion of the insertion portion 20 is described.

The second information Q2 includes, for example, the distance, journey (length along the path), direction, and path from the distal position 20a of the insertion portion 20 to the work target position (the position information regarding the affected part or lesion 501).

### (Example 1) Distance

The distance is computed from two coordinates. That is, the length of a vector computed from the difference between the coordinates is the distance.

### (Example 2) Journey (length along the path)

The journey is calculated as the length along a predesignated path when the path is predesignated and the distal position 20a of the insertion portion 20 and the work target position are on the path.

### (Example 3) Direction

The direction is computed from two coordinates. The direction is calculated as the direction of the vector to the coordinates of the work target position from the coordinates of the distal position of the insertion portion 20.

### (Example 4) Path

The path is calculated when the shape of the body cavity (lumen) M is read from the CT image as the third information Q3 and two coordinates are connected without contacting the lining of the body cavity (lumen) M on the CT image. Various paths can be taken to connect two coordinates, so that the following conditions are suitably added to the calculation to compute a path: the calculation is simple; the time of the calculation is short; and there is a little contact with the lining (lumen) of the body cavity (lumen).

The work using the endoscope 12 include, for example, the observation and diagnosis of the object by, for example, images, and treatments such as a biopsy by a treatment instrument and medical attention. In such work using the endoscope 12, information (the situation of arrival at a predetermined workable range per work) generated by the use of the first information Q1, the third information Q3, and the fourth information Q4 is the second information Q2.

FIG. 12 shows the endoscope 12 inserted in the body cavity and the situation of arrival at the workable range. A workable range 503 of the observation and diagnosis is set for the affected part or lesion 501. The second information Q2 indicates the situation of arrival of distal positions A to C of the endoscope 12 at the workable range 503. That is, the distal position A represents the situation of "deviation" from the workable range 503 set for the affected part or lesion 501. The distal position B represents the situation of "adjacency". The distal position C represents the situation of "arrival".

The changes from the distal position A to the distal position B and from the distal position B to the distal position C represent the situations of increasing adjacency.

The changes from the distal position C to the distal position B and from the distal position B to the distal position A represent the situations of increasing deviation.

The methods and procedures of generating the "coordinates of the distal positions A, B, and C of the insertion portion 20" which is the first information Q1 calculated on the basis of the sensor value SV, and the "work target position" which is the third information Q3 calculated on the basis of information K, for example, a medical record shown in FIG. 11 are as described above.

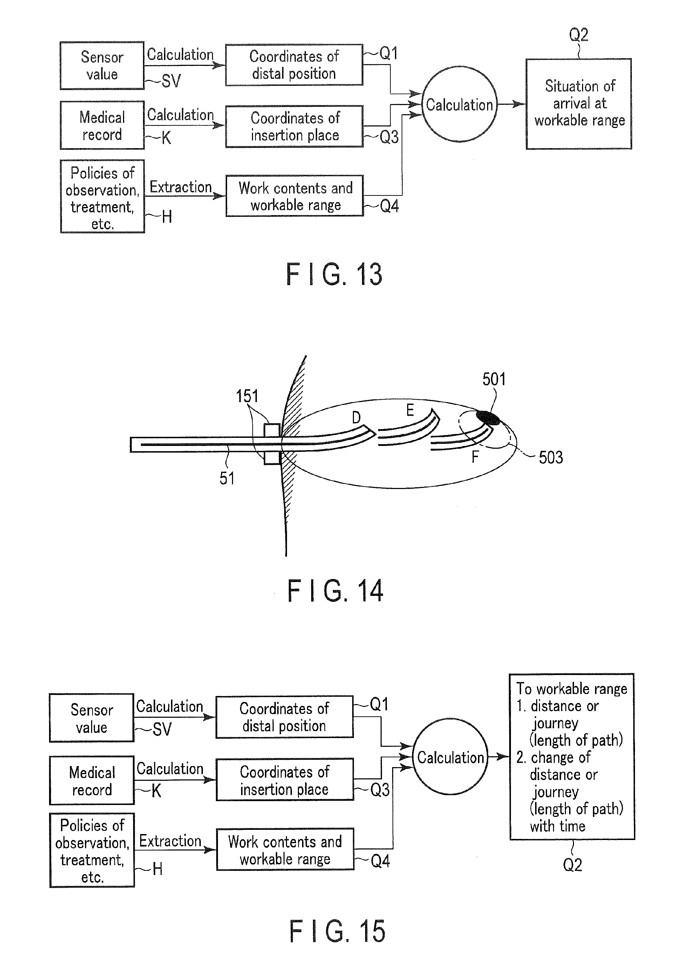

FIG. 13 shows the flow of the generation of the second information Q2 using the fourth information Q4. The same parts as the parts in FIG. 11 are denoted with the same marks and are not described in detail.

The fourth information Q4 is extracted from "policies and procedures H of observation and treatment". The fourth information Q4 includes work contents and the workable range 503 by the insertion portion 20 of the endoscope 12. When the information indicating the workable range 503 is not extracted from the fourth information Q4, the information indicating the workable range 503 is acquired by being input by a manual operation of the operator OP, by being read from the storage unit 17, or by being input from some other external device (e.g. a database).

The workable range 503 in the coordinate system provided in the body cavity M is found from the "work target position" which is the third information Q3 and from the "workable range 503 by the insertion portion 20 of the endoscope 12" which is the fourth information Q4. The "situation of arrival at the workable range 503" which is the second information Q2 can be ascertained from the relation between the workable range 503 and the coordinates of the distal position 20a of the insertion portion 20.

Specific arrival situations include the following examples:
(Example 1) "Distance and journey (length of the path) to arrive at the workable range 503,
(Example 2) Two pieces of information: the "arrival at the workable range 503" and the "location outside of the workable range 503", and
(Example 3) Information (information classified by levels on the basis of the distance and journey) such as the "adjacency to the workable range 503" and a long distance outside the workable range 503 classified by degrees of the "arrival at the workable range 503" and the "location outside of the workable range 503".

When a particular workable range is unknown or is not assumed, the positional relation between the insertion place G which is the target for the insertion of the insertion portion 20, for example, the affected part or lesion 501, and the distal position 20a of the endoscope 12 may be ascertained, and the deviation, adjacency, and arrival of the insertion portion 20 may be judged.

FIG. 14 shows the endoscope 12 inserted in the body cavity M and the situation of arrival at the workable range 503. The relation between distal positions D to F of the endoscope 12 and the workable range 503 of the observation and diagnosis is as follows:
The distal position D represents "deviation". The distal position E represents "adjacency". The distal position F represents the situation of "arrival".

The changes from the distal position D to the distal position E and from the distal position E to the distal position F represent the situations of increasing adjacency.

The changes from the distal position F to the distal position E and from the distal position E to the distal position D represent the situations of increasing deviation.

Although the situations are classified into three sections: deviation, adjacency, and arrival in the example described above, the number of classifications may be suitably set to a desirable number depending on the situation.

FIG. 15 shows the flow of the generation of the second information Q2. The same parts as the parts in FIG. 11 are denoted with the same signs and are not described in detail. The second information Q2 may include a "change of the distance and journey (length of the path) with time" to arrive at the workable range 503, and "information regarding changes of the situations of arrival, adjacency, and deviation".

As described above, the situation of arrival at the workable range 503 and the change of the situation are presented to the operator OP as the second information Q2 per work, so that the efficiency and certainty of work can be increased. A target position at which the insertion portion 20 is inserted and located in an object E, or the situation of arrival at a through-position through which the insertion portion 20 reaches the target position and the change of the situation may be presented to the operator OP as the second information Q2 instead of the workable range 503.

That is, the second information Q2 includes the work target position information indicating the target position at which the insertion portion 20 is inserted and located in an object E, or the through-position through which the insertion portion 20 reaches the target position.

The second information calculator 102 calculates, as the second information Q2, information regarding the situation of adjacency, the arrival at the predetermined range, and the stray situation of the insertion portion 20 relative to the target position or the through-position on the basis of the work target position information and the position information. Specifically, the second information calculator 102 calculates, as the second information Q2, information indicating the change of the position of the distal part of the insertion portion 20 relative to the target position or the through-position.

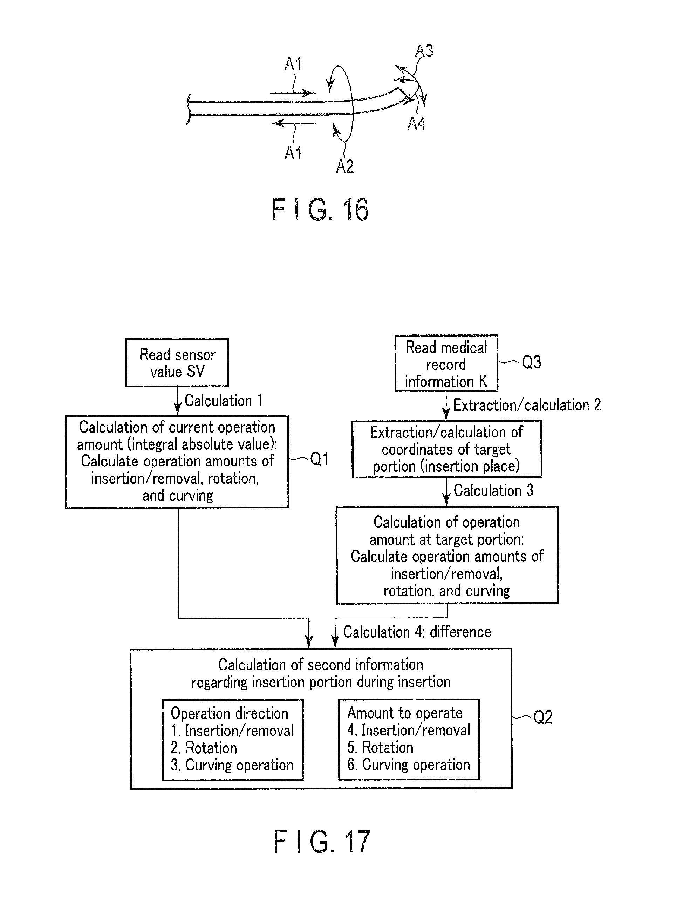

FIG. 16 shows a specific operation example of the endoscope 12. The specific operation of the endoscope 12 is as follows: An arrow A1 indicates the direction of an insertion/removal operation of the insertion portion 20. An arrow A2 indicates the direction of a rotation operation. An arrow A3 indicates the direction of, for example, the curving operation of the curving portion 23.

The insertion/removal operation is an operation to bring the insertion portion 20 in and out of the body cavity (lumen) M. The rotation operation is an operation associated with the rotation of the insertion portion 20 around the major axis. The insertion/removal operation and the rotation operation are mainly performed by the operator OP manually operating the insertion portion 20. The curving operation of the curving portion 23 is an operation performed by bending the distal side of the insertion portion 20 with, for example, the operation knobs 37LR and 37UD.

The second information Q2 includes operation instructions. The operation instructions include, for example, operation instructions for the insertion/removal operation and the rotation operation of the insertion portion 20, and an operation instruction for the curving operation of the curving portion 23. The operation instruction for the curving operation of the curving portion 23 is issued when the curving operation of the curving portion 23 is possible.

Specifically, the operation instruction as the second information Q2 includes, for example, the following information.

(Example 1) Information indicating the direction and operation amount in the distal end bending (the curving operation of the curving portion 23), rotation operation, and insertion/removal operation of the insertion portion 20.

(Example 2) Information indicating the restriction of force applied during the distal end bending (the curving operation of the curving portion 23), rotation operation, and insertion/removal operation of the insertion portion 20.

(Example 3) Information indicating the restriction of the operation speed in the distal end bending (the curving operation of the curving portion 23), rotation operation, and insertion/removal operation of the insertion portion 20.

Next, a series of processing to obtain the second information Q2 is described. A generation method described here is illustrative only, and does not show all generation methods.

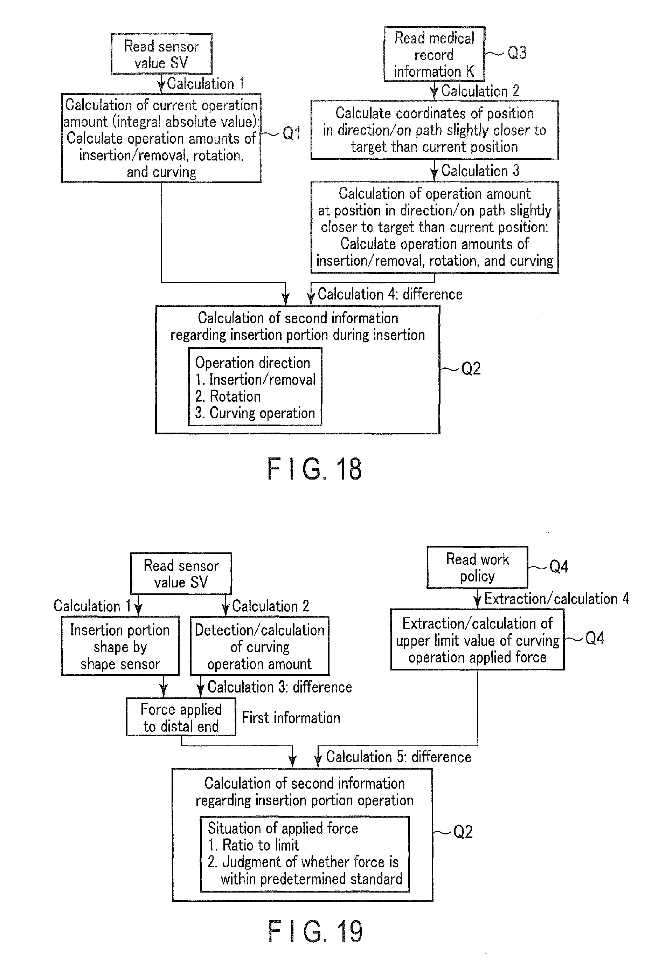

FIG. 17 and FIG. 18 show the flow of the generation of the second information Q2. In the generation of the second information Q2, an example of the generation method of the information indicating the direction and operation amount in the distal end bending operation, rotation operation, and insertion/removal operation during the insertion of the insertion portion 20 into the object is described by way of example. Regarding the operation amount of each operation, an operation situation is uniquely determined in accordance with the absolute value in the coordinate system.

### (Generation method example 1)

In the generation of the second information Q2, the first information generator 101 reads the sensor value SV, as shown in FIG. 17. On the basis of the sensor value SV, the first information generator 101 calculates, as the first information Q1, an operation amount in the current insertion situation of the insertion portion 20, and operation amounts of the insertion/removal, rotation, and curving of the distal position 20a of the insertion portion 20.

In the meantime, the second information generator 102 reads the information K, for example, a medical record from the storage unit 17 as the third information Q3. The third information Q3 is generated as the work target position which is the position information regarding the affected part or lesion 501 from the information K such as the medical record. The second information generator 102 calculates coordinates for the insertion portion 20 to arrive at the affected part or lesion 501 which is the target position on the basis of the third information Q3, and calculates an operation amount for the insertion portion 20 to arrive at the affected part or lesion 501. The operation amount of the insertion portion 20 is each of the operation amounts in the insertion/removal, rotation, and curving of the distal position 20a of the insertion portion 20.

The second information generator 102 calculates the difference between each of the operation amounts in the insertion/removal, rotation, and curving of the distal position 20a of the insertion portion 20 based on the sensor value SV and each of the operation amounts in the insertion/removal, rotation, and curving of the distal position 20a of the insertion portion 20 based on the information K such as the medical record.

The second information generator 102 calculates the second information Q2 indicating the operation to achieve the operation state at the intended target position on the basis of the difference.

In the generation of the second information Q2, the operation direction and operation amount of the insertion portion 20 are calculated. The second information Q2 includes the insertion/removal, rotation, and curving operations which are the operation directions, and the insertion/removal, rotation, and curving operations which are amounts to operate. In the generation of the second information Q2, the difference indicates the amount to operate, and the sign of the difference indicates the operation direction. The generation of the second information Q2 is effective when there is no barrier (e.g. the wall of the lumen) to the operation of the insertion portion 20 on the way to the target position.

### (Generation method example 2)

The difference of the generation of the second information Q2 between the generation method example 2 and the above generation method example 1 is described with reference to FIG. 18.

In the generation of the second information Q2, the second information generator 102 calculates coordinates at a position which is slightly moved toward the target position (intended work target position) from the current insertion position of the insertion portion 20 on the basis of the information K, for example, a medical record as the third information Q3.

The second information generator 102 calculates an operation amount at the position which is slightly moved toward the target position (intended work target position) from the current insertion position of the insertion portion 20. The operation amount of the insertion portion 20 is each of the operation amounts in the insertion/removal, rotation, and curving of the distal position 20a of the insertion portion 20.

The second information generator 102 calculates the difference between each of the operation amounts in the insertion/removal, rotation, and curving of the distal position 20a of the insertion portion 20 based on the sensor value SV (the first information Ql) and each of the operation amounts in the insertion/removal, rotation, and curving of the distal position 20a of the insertion portion 20 based on the information K such as the medical record as the third information Q3. This difference indicates the amount to operate for the slight movement.

The second information generator 102 calculates the second information Q2 indicating the operation to achieve the operation state at the target position on the basis of the difference. The second information Q2 includes the insertion/removal, rotation, and curving operations which are the operation directions. In the generation of the second information Q2, the operation directions are computed from the sign of the difference.

### (Generation method example 3)

In the generation of the second information Q2, as shown in FIG. 19, when the path to the target position (intended work target position) is known, an operation state at a position slightly closer to the target position than the current position on the path is calculated. The difference between this operation state and the current operation state is computed, and an operation to be first performed at present is calculated on the basis of the difference. In the generation of the second information Q2, the difference is the operation amount to make a slight movement. In the generation of the second information Q2, the operation directions are computed from the sign of the difference.

The three generation method examples 1 to 3 described above assume that the distal position of the insertion portion 20 is moved to the target position. Otherwise, for the generation of the second information Q2, an operation amount and an operation direction may be calculated with the target at a workable range that enables a particular operation.

FIG. 19 shows the flow of the generation of the second information Q2. In the flow of the generation of the second information Q2, a generation method of information indicating the restriction of force to be applied in the distal end bending, rotation, and insertion/removal of the insertion portion 20 during the insertion of the insertion portion 20 is described by way of example.

Force to be applied to the insertion portion 20 is calculated on the basis of the sensor value SV. In the present embodiment, the force F (FIG. 8) applied by bending the distal position 20a of the insertion portion 20 according to the method described above is calculated. That is, the second information generator 102 calculates the difference between the actual curving amount of the curving portion 23 detected by the fiber sensor 88 and the curving operation amount detected by the curving operation amount detection unit 63, and calculates the force F applied to the lining 500 of the body cavity (lumen) by the insertion portion 20 on the basis of the difference.

The force (applied force) F applied to the lining of the body cavity (lumen) M by the rotation operation or the insertion/removal operation is calculated. In the calculation of the force (applied force) F, it may be necessary to estimate the place where friction occurs in the body cavity (lumen) M and the resistance of the friction. Variations of the rotation operation or the insertion/removal operation compared to the force F or torque that is actually applied are calculated on the basis of the sensor value SV. That is, the first information generator 101 calculates each of the operation amounts (the first information Q1) in the insertion/removal, rotation, and curving of the distal position 20a of the insertion portion 20 based on the sensor value SV. When the first information Q1 is calculated, frictional resistance and frictional force are calculated on the basis of the first information Q1.

In the meantime, the value (upper limit value) of the applied force F is acquired for the force F applied to the wall of the body cavity (lumen) M. The value (upper limit value) of the applied force is a value at which a temporary load (e.g. pain) or a damage can occur. The value (upper limit value) of the applied force is acquired by extracting or calculating from the fourth information Q4. The fourth information Q4 is set from the perspective of a load on the object, and includes information indicating the upper limit value of the force F applied to the object by the insertion portion 20.

When the value (upper limit value) of the applied force F is acquired, the second information calculator 102 compares the value (upper limit value) of the applied force F with the force (applied force) F applied to the lining of the body cavity (lumen) M. As a result of this comparison, the second information calculator 102 calculates, as the second information Q2, the situation of whether the force (applied force) F is within a predetermined ratio to the value (upper limit value) of the applied force F or within a predetermined standard. The second information Q2 includes information indicating the relation between the upper limit value of the applied force F and the force F actually applied to the object by the insertion portion 20.

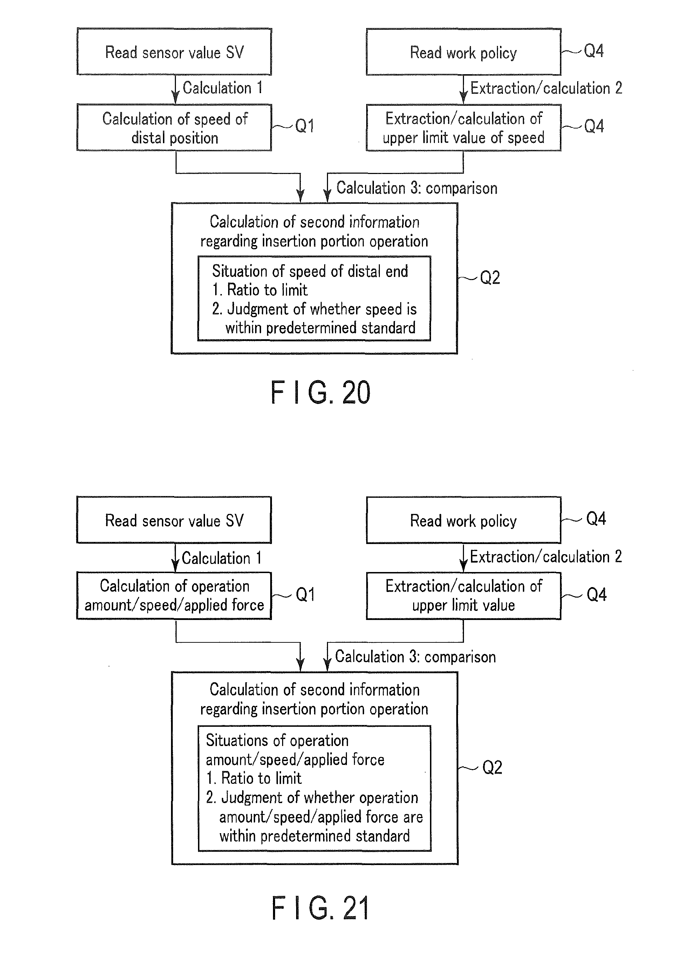

FIG. 20 shows the flow of the generation of the second information Q2. A generation method of information indicating the restriction of the speed of the distal end bending, rotation, and insertion/removal during the insertion of the insertion portion 20 is described by way of example.

The first information generator 101 calculates the speed of the insertion portion 20, for example, the speed of the distal end of the insertion portion 20 on the basis of the sensor value SV. Specifically, the speed of the insertion portion 20 is calculated by the temporal differentiation of the position of the distal end of the insertion portion 20 after the calculation of the position of the distal end of the insertion portion 20.

The controller 19 sets the upper limit value of the speed of the insertion portion 20 as the fourth information Q4. The fourth information Q4 is the upper limit value of the speed at which a temporary load (e.g. pain) or a damage is given to the object, for example, in the event of the collision of the insertion portion 20 with the lining of the body cavity (lumen) M when a trouble occurs in the execution of work such as an observation or a treatment.

The second information calculator 102 compares the degree of the speed of the insertion portion 20 which is the first information Q1 with the upper limit value of the speed of the insertion portion 20 which is the fourth information Q4, and calculates, as the second information Q2, the ratio of the speed of the insertion portion 20 to the limit (upper limit value) or the situation of whether the speed of the insertion portion 20 is within a predetermined standard.

FIG. 21 shows the flow of the generation of the second information Q2. The flow of the generation of the second information Q2 is an example of the application of the generation method of the second information Q2 shown in FIG. 20 to the three: the operation amount, the speed, and the application force.

In this example, the upper limit values of the operation amount, the speed, and the application force are set so that a desired operation can be stably and certainly performed without temporarily or permanently damaging the object. In other words, the upper limit values of the operation amount, the speed, and the application force are set to eliminate the uncertainty of the operation.

In this example, the upper limit values are set for the operation amount, the speed, and the application force. However, for example, when the directions of the operation, movement, and applied force are specified, lower limit values may be further set, and whether the operation amount, the speed, and the application force are between the upper limit values and the lower limit values may be judged.

When an operation, movement, or applied force equal to or more than a given level is required in specific work, a lower limit value may be only set and whether the operation, movement, or applied force is equal to or more than the lower limit value, or a lower limit value and an upper limit value may be set and whether the operation, movement, or applied force is between the lower limit value and the upper limit value may be judged.

As the second information Q2, whether the operation, movement, or applied force is within the ratio to the limit (the upper limit value, the lower limit value) or a predetermined standard may be judged, and warning information may be properly generated on the basis of the judgment result. The target for the generation of the warning information includes the operation amount, the speed, and force applied to the body cavity by the insertion portion 20 for the curving operation, rotation operation, or insertion/removal operation. The methods described above can be used for the calculation methods of these values.

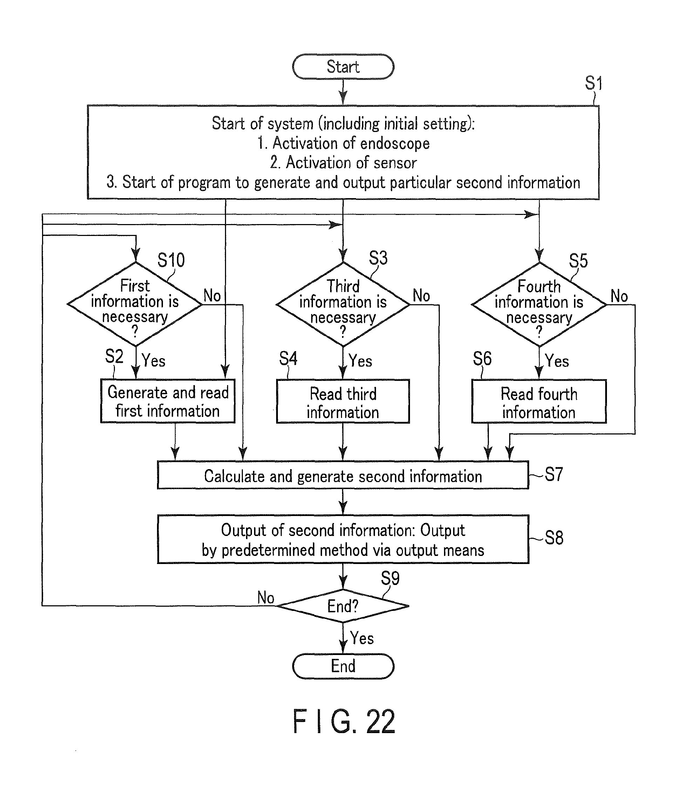

Next, a series of processing associated with the output of the second information Q2 by the present system 10 is described with reference to a flowchart shown in FIG. 22.

When the present system 10 is operated for activation, the controller 19 performs initial setting for acquiring the first information Q1 (step S1). That is, the controller 19 activates the endoscope 12, starts a program (application) for generating and outputting the second information Q2, and activates each of the sensors 101a (the curving operation amount detection unit 63, the fiber sensor 88, and the insertion portion sensor 13. In other words, in step S1, the controller 19 starts the program for obtaining the second information Q2, performs initial setting of the endoscope 12, and reads information necessary for the generation of the second information Q2. In step S1, the controller 19 activates the first information generator 101, and activates the output unit 16 for displaying and outputting the second information Q2.

The second information calculator 102 causes the first information generator 101 to generate the first information Q1, and reads the first information Q1 (step S2).

The second information calculator 102 judges whether the third information Q3 is necessary for the generation of the second information Q2 (step S3). If it is judged that the third information Q3 is necessary (YES), the second information calculator 102 reads the third information Q3 from the storage unit 17 (step S4). If the third information Q3 is not necessary (NO), the second information calculator 102 moves to step S7.

Furthermore, the second information calculator 102 judges whether the fourth information Q4 is necessary for the generation of the second information Q2 (step S5). If it is judged that the fourth information Q4 is necessary (YES), the second information calculator 102 reads the fourth information Q4 from the storage unit 17 (step S6). If the fourth information Q4 is not necessary (NO), the second information calculator 102 moves to step S7.

If necessary processing in step S2 to step S6 is finished, the second information calculator 102 generates the second information Q2 (step S7). The specific generation method of the second information Q2 is as has been described above.

After having generated the second information Q2, the second information calculator 102 outputs the second information Q2 to the output unit 16 (step S8).

The second information calculator 102 judges whether to finish the generation of the second information Q2 (step S9). If it is judged that the generation of the second information Q2 is not finished, the second information calculator 102 branches into steps S3, S5, and S10. If the generation of the second information Q2 is finished, the second information calculator 102 finishes the processing in this flowchart.

The second information calculator 102 judges in step S10 whether new first information Q1 is necessary for the generation of the second information Q2. If it is judged that the new first information Q1 is necessary, the second information calculator 102 moves to step S2. If the new first information Q1 is not necessary, the second information calculator 102 moves to step S7.

In the generation of the second information Q2, the second information calculator 102 always reads at least one of the third information Q3 and the fourth information Q4.

The second information calculator 102 may process step S2 and step S3 in parallel. The second information calculator 102 may process step S4 and each of steps S5 and S6 in parallel.

The generation of the first information Q1 by the first information generator 101 may be separately repeated independently of the reading of the first information Q1 in step S2.

As described above, according to the present first embodiment, it is possible to provide, by proper timing, not only the sensor information but also the support information useful for the operator OP as the information regarding the insertion portion 20 in the object insertion system 10 applied to, for example, the endoscopic system.

The calculation processing of the second information Q2 by the second information calculator 102 specifically includes, for example, the following processing.

Position information indicating the relative positions of the object and the distal part of the insertion portion 20 is used as the first information Q1. The work target position information indicating the target position of work in the object is used as the third information Q3.

The second information calculator 102 calculates, as the second information Q2, work target relative position information indicating the positional relation between the distal part of the insertion portion 20 and the work target position on the basis of the position information and the work target position information.

As another example, the first information Q1 uses the position information indicating the relative positions of the object and the distal part of the insertion portion 20. The work target position information indicating the target position of the work in the object is used as the third information Q3.

The second information calculator 102 calculates work target relative position information indicating the relative positions of the distal part of the insertion portion 20 and the work target position on the basis of the position information and the work target position information. The second information calculator 102 selects the third information Q3 and the fourth information Q4 used for the calculation of the second information Q2 on the basis of the work target relative position information, and sets a calculation used to figure out the second information Q2.

While the present invention has been described above in connection with the first embodiment, it should be understood that the present invention is not limited to the first embodiment described above, and for example, various modifications and applications can be made within the spirit of the present invention as follows:

### [Second Embodiment]

Next, an object insertion system according to a second embodiment of the present invention is described. In the present second embodiment, the differences between the object insertion systems 10 according to the first and second embodiments are described to avoid the repetition of explanations, and repeated explanations are omitted. The same components are denoted with the same signs and are not described.

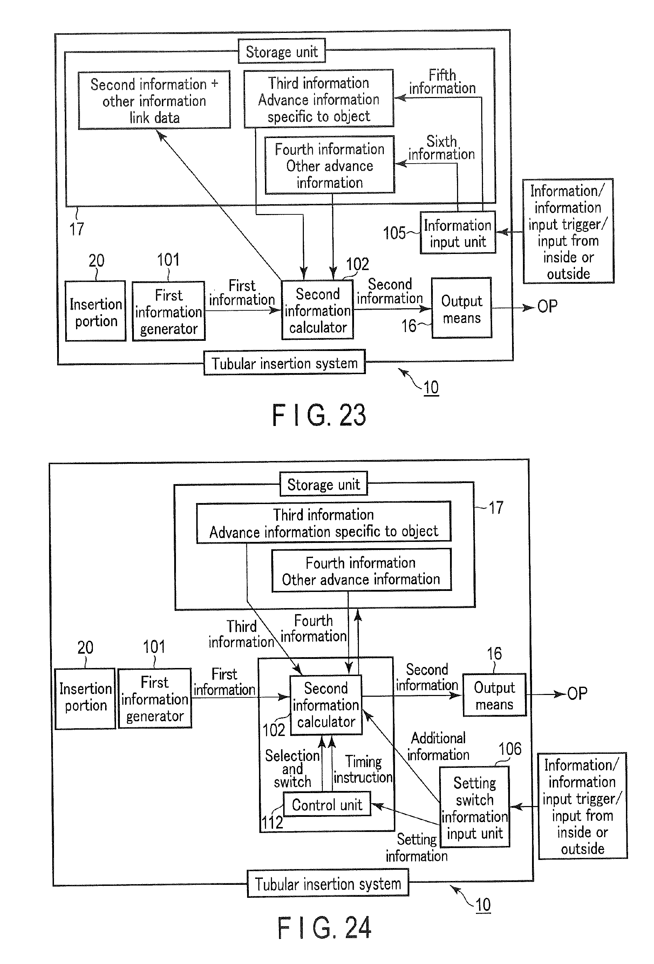

FIG. 23 shows one configuration example of the object insertion system 10 according to the second embodiment of the present invention. The present object insertion system 10 includes an information input unit 105 in addition to the object insertion system 10 according to the first embodiment described above.

The present object insertion system 10 can properly update the third information Q3 and the fourth information Q4 stored in the storage unit 17 on the basis of fifth information Q5 and sixth information Q6 input from the information input unit 105.

The information input unit 105 sequentially inputs the fifth information Q5 based on newly obtained diagnosis and treatment results and the sixth information Q5 indicating added and corrected policies and procedures of work (diagnosis and treatment) during the work (e.g. an insertion operation) by the operator OP.

In other words, the new fifth information Q5 regarding the object is input to the information input unit 105 after the start of the work (fifth information input unit). The sixth information Q6 which is new information regarding the work is input to the information input unit 105 after the start of the work (sixth information input unit).

The fifth information Q5 and the sixth information Q6 may be directly input to the information input unit 105 by the operator OP, may be automatically loaded from, for example, an internal/external device or database having the fifth information/sixth information, or may be loaded on the basis of an information input trigger from the operator OP.

The fifth information Q5 input to the information input unit 105 is sent to the storage unit 17, and additionally written to the third information Q3 indicating the diagnosis and treatment results prestored in the storage unit 17.

The second information calculator 102 generates the second information Q2 using, for the calculation, the third information Q3 updated on the basis of the fifth information Q5 during the work. As a result, the second information Q2 that reflects new information based on the observation and treatment obtained during the work can be provided.

The sixth information Q6 input to the information input unit 105 is sent to the storage unit 17, and additionally written to the fourth information Q4 indicating the policies and procedures of the work (diagnosis and treatment) prestored in the storage unit 17.

The second information calculator 102 generates the second information Q2 using, for the calculation, the fourth information Q4 updated on the basis of the sixth information Q6 during the work. As a result, the second information Q2 that reflects the information regarding the new policies and procedures determined during the work can be provided.

The object insertion system 10 stores the second information Q2 in the storage unit 17 in association with the first information Q1, the third information Q3, and the fourth information Q4. In FIG. 23, "second information + other information link data" is written in the storage unit 17 to show the association of the second information Q2 with the first information Q1, the third information Q3, and the fourth information Q4.

Specifically, for example, each piece of the second information Q2 can be combined with corresponding information and the combined information can be saved in the storage unit 17. The pieces of information ranging from the first information Q1 to the fourth information Q4 are classified and saved in the storage unit 17, and a link of each piece of information which pairs each piece of second information Q2 may be manageably saved.

The second information Q2 is thus properly saved in association with pieces of information ranging from the first information Q1 to the fourth information Q4, so that it is possible to track how the second information Q2 has been generated. Consequently, it is possible to reproduce via the output unit 16 or analyze the situations during insertion and work and the timing of providing the second information Q2. The information in which the second information Q2 is associated with each piece of information can be provided to, for example, another database.

The second information Q2 calculated by the second information calculator 102 may be stored in the storage unit 17 in association with the third information Q3 and/or the fifth information Q5 as well as the fourth information Q4 and/or the sixth information Q6.

As described above, according to the present second embodiment, advantageous effects similar to those of the object insertion system 10 according to the first embodiment described above are provided, and the third information Q3 and the fourth information Q4 are updated as needed, so that the diagnosis and treatment results and the policies and procedures of the work (e.g. diagnosis and treatment) can be updated, and a basic function as an electronic medical record can be provided accordingly.

### [Third Embodiment]

Next, an object insertion system according to a third embodiment of the present invention is described. In the present third embodiment, the differences between the object insertion systems 10 according to the first and third embodiments are described to avoid the repetition of explanations, and repeated explanations are omitted. The same components are denoted with the same signs and are not described.

A switch function related to the second information Q2 is added to the object insertion system 10 according to the present third embodiment.

FIG. 24 shows one configuration example of the object insertion system according to the third embodiment of the present invention. The object insertion system 10 includes a setting switch information input unit 106, and a control unit 112 which controls the switch of the second information Q2, in addition to the object insertion system 10 according to the first embodiment described above. The second information calculator 102 and the control unit 112 are associated in their processing contents, and may therefore be provided as one component for higher-speed processing and compactness.

Setting information indicating setting regarding the calculation of the second information Q2 by the second information calculator 102 is input to the setting switch information input unit 106.

The control unit 112 performs control regarding the selection and/or switch of the combination of information required for the calculation of the second information Q2 by the second information calculator 102 on the basis of the input setting information.

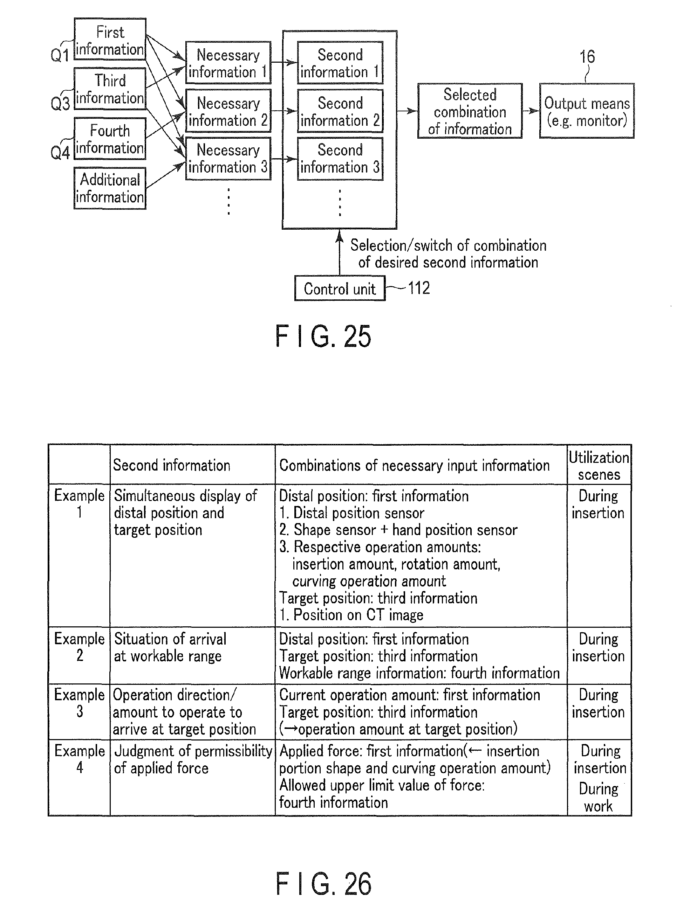

FIG. 25 shows the concept of the switch control associated with the second information Q2 by the control unit 112.

The control unit 112 selects and switches the second information Q2 to be generated, selects and switches the input information necessary for the generation of the second information Q2, selects and switches an output method by the output unit 16, and selects and switches an optimum computation algorithm for the generation of the second information Q2, in accordance with the setting information input from the setting switch information input unit 106.

Furthermore, the control unit 112 determines output timing of the second information Q2 on the basis of the first information Q1. The control unit 112 performs setting on the basis of information regarding initial setting stored in the storage unit 17 and the setting information input from the setting switch information input unit 106. When the second information Q2 is selected and switched, necessary input information is selected and switched at the same time.

The importance of the second information Q2 may significantly vary according to the operator OP. The operator OP may only desire the second information Q2 that enhances insertability in particular, or may only desire the second information Q2 associated with work such as an observation or a treatment. It may be considered unnecessary for an experienced operator OP to provide information other than the warning information. If the contents of the second information Q2 to be required for each work target significantly vary or if the body cavity (lumen) M or the kind of work target changes, the change can further increase.

The above-mentioned selection and switch may be automatically performed by the control unit 112 on the basis of, for example, object, the third information (e.g. information indicating the lumen of the work target) Q3 and the fourth information (e.g. information indicating the work contents) Q4. An operation by the operator OP may be received by the setting switch information input unit 106 to perform desired selection and switch.

The second information Q2 that is needed may vary from user to user. In this case, the user may be able to select desired second information Q2 so that the information necessary for the user can be only provided.

Specifically, combinations of information shown in FIG. 26 can be taken shown as examples. FIG. 26 shows examples of setting information in which the second information Q2, a combination of input information necessary for the generation of the second information Q2, and utilization scenes of the second information Q2 are associated.

In "Example 1", "simultaneous display of the distal position of the insertion portion 20 and the target position" is shown as the "second information Q2", the "distal position of the insertion portion 20" is shown as "the first information Q1 regarding the combination of input information necessary for the generation of the second information", "the target position" is shown as the "the third information regarding the combination of input information necessary for the generation of the second information", and "during insertion" is shown as the "utilization scene".

The "distal position of the insertion portion 20" is acquired by
(1) a sensor provided at the distal position of the insertion portion 20,
(2) the shape sensor (fiber sensor 88) and a hand position sensor (insertion portion sensor 13), and
(3) each operation amount (the insertion amount, the turning amount, and the curving operation amount).
The "target position" is acquired by the "position on the CT image".

In "Example 2", "the situation of arrival at the workable range" is shown as the "second information", the "distal position of the insertion portion 20" is shown as "the first information regarding the combination of input information necessary for the generation of the second information", "the target position" is shown as the "the third information regarding the combination of input information necessary for the generation of the second information", "workable range information" is shown as "the fourth information regarding the combination of input information necessary for the generation of the second information", and "during insertion" is shown as the "utilization scene".

In "Example 3", "the operation direction/amount to operate to arrive at the target position" are shown as the "second information", the "current operation amount" is shown as "the first information regarding the combination of input information necessary for the generation of the second information", "the target position" is shown as the "the third information regarding the combination of input information necessary for the generation of the second information", and "during insertion" is shown as the "utilization scene".

In "Example 4", "judgment of the permissibility of force applied to the object" is shown as the "second information", "force being applied to the object" is shown as "the first information regarding the combination of input information necessary for the generation of the second information", "the upper limit value of the force applied to the object" is shown as "the fourth information Q4 regarding the combination of input information necessary for the generation of the second information", and "during insertion and during work" are shown as the "utilization scenes".

For example, the operator OP skilled in operation selects the output of the "simultaneous display of the distal position and the target position" as the second information Q2, as in "Example 1". In other words, the operator OP skilled in operation does not require (does not select) the output of "the operation direction/amount to operate to arrive at the target position" as the second information Q2 in "Example 3".

As a result of such a selection, the "simultaneous display of the distal position and the target position" is output to the output unit 16. The operator OP can perform the insertion operation while judging the operation method and operation amount referring to the above output.

Thus, the operator OP skilled in operation requires no aid for the specific operation direction and operation amount. Therefore, "the operation direction/amount to operate to arrive at the target position" in "Example 3" is inconvenient information. Unuseful information is thus not output (displayed), so that the operator OP is not distracted, and the operator OP can concentrate on the insertion operation.

For example, the operator OP can select, as the second information Q2, the output of the "simultaneous display of the distal position and the target position" in "Example 1" and the "judgment of the permissibility of applied force" in "Example 4". During the insertion into the target position, the "simultaneous display of the distal position and the target position" can be output as the second information Q2. During the work, the "judgment of the permissibility of applied force" can be output as the second information Q2.

At the time of the switch from the insertion to the work, "the situation of arrival at the workable range" in "Example 2" can be used. "The situation of arrival at the workable range" in "Example 2" is not used as the second information Q2 to be output to the outside, and does not always need to be output.

As in the present third embodiment, "during insertion" and "during work" are separated, and the second information Q2 presented by the timing of the switch of the operation purpose is switched from the information for insertion to the information for work, so that the second information Q2 suited to each use scene alone can be provided to the operator OP.

Thus, "the situation of arrival at the workable range" which is the second information Q2 generated from the first information Q1, the third information Q3 and the fourth information Q4 (generated by the second information calculator 10 is used, so that it is no longer necessary to separately perform a calculation to know proper switch timing.

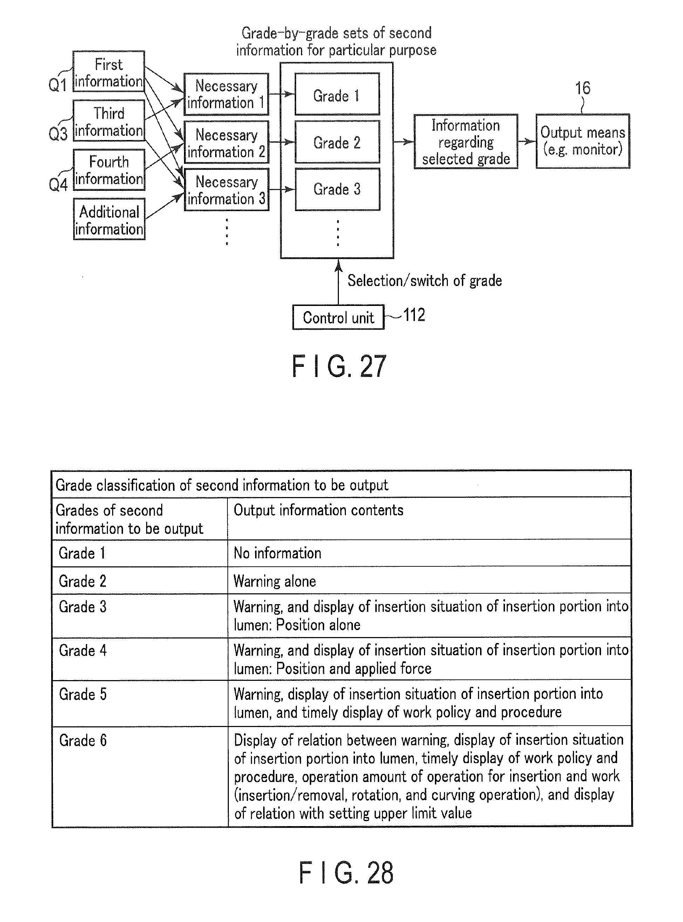

FIG. 27 shows the concept of switch control associated with the second information Q2 by the control unit 112. In the example shown in FIG. 27, "grades" are set for the selection and switch of the second information Q2. The control unit 112 controls the selection and switch of the second information Q2 on the basis of the grades.

FIG. 28 shows an example of grade classification associated with the selection and switch of the second information Q2. In the selection and switch of the second information Q2, the grades are information for switching the second information Q2 output by the output unit 16 (provided to the operator OP). FIG. 28 shows six grades "1" to "6" corresponding to information providing levels desired by the operator OP. The grade "1" is a grade in which the second information Q2 is not output (provided to the operator OP). The grade "6" is a grade in which the second information Q2 is output (provided to the operator OP) to the fullest.

These grades are set for each operator OP on the basis of the degree at which the operator OP needs the second information Q2, for example, on the basis of the degree at which the operator OP is skilled in an operative method. The operator OP selects a desired grade. The control unit 112 controls the second information calculator 102, and outputs (provides) the second information Q2 on the basis of the grade selected by the operator OP.

The "grade 1" indicates "no information (no output of the second information)".

The "grade 2" indicates a "warning alone information (output of information regarding a warning alone among the second information)".

The "grade 3" indicates "display of a warning and the insertion situation (position alone) of the insertion portion 20 into the lumen alone".

The "grade 4" indicates "display of a warning and the insertion situation (position and applied force) of the insertion portion 20 into the lumen".

The "grade 5" indicates a "warning, display of the insertion situation of the insertion portion 20 into the lumen, and timely display of the work policy and procedure".

The "grade 6" indicates a "warning, display of the insertion situation of the insertion portion 20 into the lumen, timely display of the work policy and procedure, the operation amounts of the operations (insertion/removal, rotation, and curving operation) for insertion and work, and display of the relation with the setting upper limit value".

The grades are set so that the presented contents of the second information Q2 are enriched by stages as the level rises from "1" to "6". The six grades "1" to "6" are provided, so that it is possible to adapt to wide range of operators OP from the operator OP who needs no support information to the operator OP who needs support information that can be provided by full specification. The operator OP has only to select a grade including information which seems to be necessary for the operator OP (selection is also easy), and can be easily provided with proper support information.

Selection methods of the grades include a method by which the operator OP directly selects a grade from a menu screen on, for example, the monitor, and a method by which the operator OP selects an index indicating the experience and ability to indirectly select a grade. For example, some of information regarding the procedural skill level and past work experience of the operator OP, and, for example, the matured level of the procedure/diagnosis/treatment of a particular skill to be the number of times of trainings, the number of times of procedures, and an index is associated with each of the grades "1" to "6". The operator OP inputs and selects the information, and can thereby select the grades "1" to "6" associated with this information.

According to this selection method of the grades, the operator OP can enjoy the convenience attributed to the selection and switch of the second information Q2 that uses the grades and can easily make full use of the grades even the operator OP does not know the meaning of each grade.

The grades may be set so that the output (providing) level of the second information Q2 is changed for each work such as insertion or treatment. The grades may be automatically switched, for example, when wrong operations and warnings frequently occur.

A mode for training may be set, and in this mode for training, for example, a voice guidance to encourage learning that is not normally performed may be carried out before the patient who is the object, or the acceptability of the result of insertion and work and the level judgment result may be output.

The generation and output of the second information Q2 are optimized in accordance with, for example, the experience and skill of the user, so that the efficiency, certainty, and safety of the work can be improved.

The timing of the output (provision) of the second information Q2 may be set on the basis of the position of the insertion portion 20 of the endoscope 12. For example, when the insertion portion 20 of the endoscope 12 is located in a particular position or range, the control unit 112 controls the second information calculator 102 to output the second information Q2 that is important or effective in the particular position or range by this timing.

Specifically, the control unit 112 basically provides, for example, the second information Q2 regarding insertion until the intended work target position or workable range is reached, and finishes the output of the second information Q2 at the point where the work target position or workable range is reached.

The control unit 112 has only to control the second information calculator 102 to output (provide), as the second information Q2, "information of arrival" or "precautions and past information regarding the intended work target" associated with the position which is the first information Q1, and the "work contents and work policy to be performed for the work target" when the part which is the entrance of a particular organ and which is the reference of the work, for example, the entrance of the stomach or bladder or the intended work target position or workable range is reached.

The information indicating a particular position or range regarding the setting of timing is stored in the storage unit 17 as the fourth information Q4.

The second information calculator 102 combines the fourth information Q4 with the first information Q1 to generate timing. Information indicating a particular position or range regarding the setting of timing may be provided to the setting switch information input unit 106 as additional information instead of the fourth information Q4. Moreover, if a particular position or range regarding the timing of the output of the second information Q2 is judged by the operator OP, a trigger may be given to the setting switch information input unit 106 by the judged timing.

The trigger is input to the setting switch information input unit 106, and the setting switch information input unit 106 performs the selection and switch of the second information Q2 generated in accordance with the setting information, the selection and switch of the input information necessary for the generation of the second information Q2, the selection and switch of the output method by the output unit 16, and the selection and switch of the optimum computation algorithm for the generation of the second information Q2.

The effective second information Q2 is timely output (provided to the operator OP) by particular timing, so that the efficiency, certainty, and safety of the work can be improved.

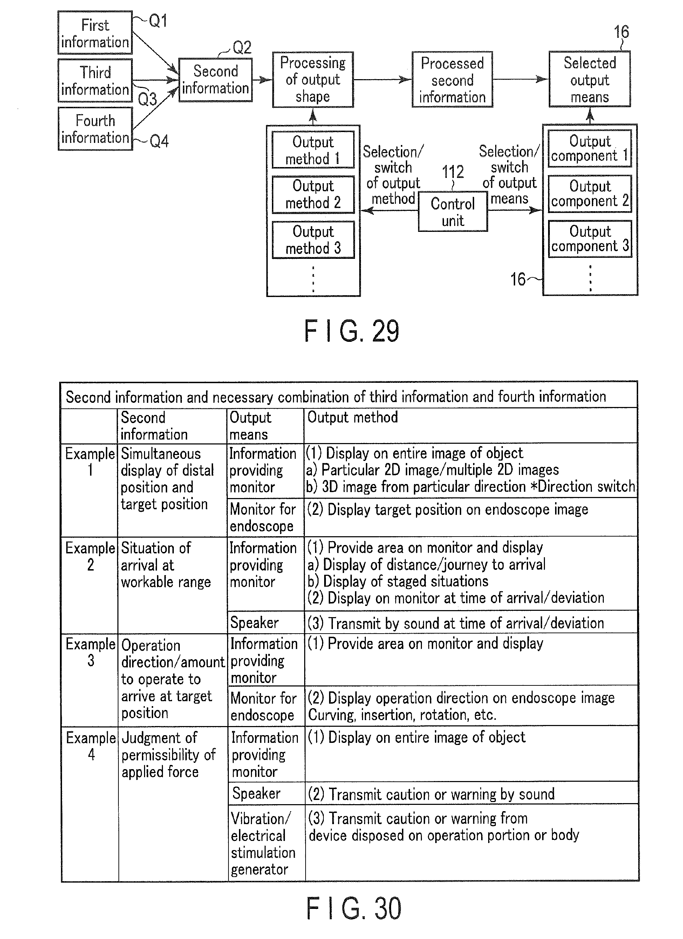

The output by the output unit 16 may be switched. FIG. 29 shows the concept of switch control associated with the second information Q2 by the control unit 112. In this example, multiple output components are provided as the output unit 16. The control unit 112 controls the selection and switch as to which of the output components to use as the output unit 16 or which output method to use.

The output components constituting the output unit 16 specifically include, for example, an information providing monitor, a monitor for endoscope images, a speaker, and a vibration/electrical stimulation generator.

In the selection and switch control by the control unit 112 in this example, the warning display may be switched from the display on the information providing monitor to the vibration stimulation generator (vibration device) provided in the operation portion of the endoscope 12.

The output method includes, for example, the switch between characters and graphics, the display size, the switch of display places, and the switch of colors if the information providing monitor is taken as an example. Moreover, the output method includes the repetition frequency of outputs and the switch between the presence and absence of output.

According to this output switch, the whole object insertion system 10 is easier for the operator OP to use. The output unit 16 optimum for each piece of second information Q2 can be selected. Information that seems important to the user can stand out.

Next, a specific example of output switch is described with reference to FIG. 30.

FIG. 30 shows examples of setting information regarding output switch in which the second information Q2, output means, and output methods are associated. In "Example 1", one of the information providing monitor and the monitor for endoscope can be selected and switched as the output unit 16 for the simultaneous display of the distal position (the distal position of the insertion portion 20) and the target position which is the second information Q2.

One of the output methods can be selected and switched in accordance with the above selection and switch: display on the entire image of the object (a; a particular 2D image/multiple 2D images, b; a 3D image from a particular direction), and display of the target position on an endoscope image.

According to "Example 1", the 2D (two-dimensional) image display and the 3D (three-dimensional) image display can be switched in the "information providing monitor". In the monitor for endoscope, the target position is displayed on an object lumen image which is the endoscope image. The output means and the output methods can be thus selected, so that an image medium and a display method that the operator OP prefer can be selected, which leads to improvements in the efficiency and quality of insertion and work. Display optimum for the operation contents can be performed by switching the output means or the output method for each content of insertion of an endoscope or and work using an endoscope.

In "Example 2", one of the "information providing monitor" and the "speaker" can be selected and switched as the output unit 16 for "the situation of arrival at the workable range" which is the second information Q2. One of the output methods can be selected and switched in accordance with the above selection and switch: "display in an area provided on the monitor (a; display of distance/journey to arrival, b; display of staged situations)", and "speech communication at the time of arrival/deviation".

According to "Example 2", even when the "information providing monitor" is used to output "the situation of arrival at the workable range" as the second information Q2, there are multiple display methods: the "display of distance/journey to arrival", the "display of staged situations", and the "display on the monitor at the time of arrival/deviation alone".

When the "speaker" is used, the situation can be reported by voice or electronic sound at the time of arrival at the workable range or the target position or at the time of deviation. The operator OP selects and sets from among the above options, so that the second information which ensures the preference of the operator OP and the certainty of information transmission can be output (provided).

In "Example 3", one of the "information providing monitor" and the "monitor for endoscope" can be selected and switched as the output unit 16 for "the operation direction/the amount to operate for arrival at the target position" which is the second information Q2. One of the output methods is selected and switched: "display in an area provided on the monitor" and "display of the operation direction (e.g. curving, insertion, and rotation) on the endoscope image" in accordance with the above selection and switch.

According to "Example 3", the "information providing monitor" and the "monitor for endoscope" can be selected and switched as the output unit 16 for the second information Q2: "the operation direction/the amount to operate for arrival at the target position". An area is provided to display each operation in the "information providing monitor".

In the "monitor for endoscope", the operation direction is displayed on the object lumen image which is the endoscope image. In the "information providing monitor", the operation direction and a specific operation amount can be displayed for each operation, which is particularly advantageous when the amount of operation to be needed is important.

When the operation direction is displayed on the object lumen image, the operation direction is superimposed and displayed on the lumen image which the operator OP mainly stares, so that the operator OP can ascertain the operation direction without moving eyes.

The operator OP thus selects and sets from among various options, so that output switch which is easily ascertained by the operator OP and which is adapted to the work contents can be performed.

In "Example 4", one of the "information providing monitor", the "speaker", and the "vibration/electrical stimulation generator" can be selected and switched as the output unit 16 for the "judgment of the permissibility of force applied to the object" which is the second information Q2. One of the output methods can be selected and switched in accordance with the above selection and switch: "display on the entire image of the object", "transmit a caution or warning by sound", and "transmit a caution or warning from a device disposed on the operation portion or the body".

According to "Example 4", one of the "information providing monitor", the "speaker", and the "vibration/electrical stimulation generator" can be selected and switched as the output unit 16 for the "judgment of the permissibility of applied force" which is the second information Q2. In the display on the "information providing monitor", the magnitude and direction of force and the place to which the force is applied are displayed. When the "speaker" is used, warning sound is produced, or a speech warning is given.

For example, the "information providing monitor" is used as the output unit 16 for the operator OP watching the "information providing monitor". When the operator OP is not watching the "information providing monitor" or there are moments in which the operator OP is not looking at the monitor, or when there is another operator OP who is not watching the "information providing monitor", the "speaker" is used to transmit warning sound or warning contents by sound.

The "vibration/electrical stimulation generator" is attached to, for example, the operation portion 30 or the operator OP, and directly transmits a stimulus to the body of the operator OP, for example, in the event of a warning. Thus, for example, even when the operator OP is devoted to work and tends to be unaware of the warning or when an instantaneous response is needed after the issuance of a warning, the operator OP can be caused to take the highest priority to understanding the warning, to immediately stop work, or to cope with the warning.

For example, it is possible to attach a flag indicating the urgency or importance. In accordance with the flag, the control unit 112 controls the calculation processing of the second information Q2 by the second information calculator 102 and the switch processing of the output components and the output methods for outputting the second information Q2.

As described above, according to the present third embodiment, advantageous effects similar to those of the object insertion system according to the first embodiment described above are provided, and it is possible to provide an object insertion system which can provide necessary operation support information to the operator OP by the timing optimum for each operator OP.

Furthermore, the embodiments described above include various stages of inventions, and various inventions can be extracted by properly combining the disclosed requirements. For example, when the problems described in the section Technical Problem can be solved and advantageous effects described in the section Advantageous Effects of Invention can be obtained even if some of all the requirements shown in the embodiments are eliminated, a configuration in which those requirements are eliminated can also be extracted as an invention.

## Claims

1. An object insertion system **characterized by** comprising:
an insertion portion which is inserted into a body cavity of an object to perform predetermined work;
a first information generator which generates first information, the first information being information indicating at least one of an insertion state and an operation state of the insertion portion in the body cavity;
a storage unit which stores at least one of third information regarding the object and fourth information associated with the work;
a second information calculator which calculates second information based on the first information and at least one of the third information and the fourth information, the second information being operation support information to insert the insertion portion and perform the predetermined work; and
an output unit which outputs the second information.

2. The object insertion system according to claim 1, **characterized in that** the first information includes information indicating a shape of the insertion portion, or information indicating relative positions of the insertion portion and the object.

3. The object insertion system according to claim 2, **characterized in that** the information indicating the relative positions of the insertion portion and the object includes information indicating at least one of an insertion amount and a turning amount of the insertion portion relative to the object, or information indicating a contact state between the insertion portion and the object.

4. The object insertion system according to claim 1, **characterized in that** the third information includes information specific to the object.

5. The object insertion system according to claim 4, **characterized in that** the information specific to the object includes information indicating a medical history of the object, or information indicating past diagnostic results of the object.

6. The object insertion system according to claim 1, **characterized in that** the fourth information includes information indicating a policy of the work, information indicating a procedure of the work, information indicating a specification of an instrument associated with work, information indicating a performance of the instrument associated with work, or advance information regarding the work.

7. The object insertion system according to claim 1, **characterized in that**
the first information includes position information indicating relative positions of the object and a distal part of the insertion portion,
the third information includes work target position information indicating a target position of the work in the object, and
the second information calculator calculates, as the second information, work target relative position information indicating relative positions of the distal part of the insertion portion and the target position of the work based on the position information and the work target position information.

8. The object insertion system according to claim 1, **characterized in that**
the first information includes position information indicating relative positions of the object and a distal part of the insertion portion,
the third information includes work target position information indicating a target position of the work in the object, and
the second information calculator calculates work target relative position information indicating relative positions of the distal part of the insertion portion and the target position of the work based on the position information and the work target position information, selects the third information and the fourth information used for a calculation of the second information, and sets a calculation used to compute the second information based on the work target relative position information.

9. The object insertion system according to claim 1, **characterized in that**
a workable range which permits a distal part of the insertion portion to do the work is set, and
the second information includes information regarding a workable range arrival situation indicating positional relation of the distal part of the insertion portion to the workable range.

10. The object insertion system according to claim 1, **characterized by** further comprising
a fifth information input unit to which fifth information that is new information regarding the object is input after a start of the work,
wherein the storage unit updates the third information by the fifth information, and
the second information calculator uses updated third information to calculate the second information.

11. The object insertion system according to claim 1, **characterized by** further comprising
a sixth information input unit to which sixth information that is new information regarding the work is input after a start of the work,
wherein the storage unit updates the fourth information by the sixth information, and
the second information calculator uses updated fourth information to calculate the second information.

12. The object insertion system according to claim 1, **characterized in that** the second information includes an instruction to insert the insertion portion into the object, or information indicating an operation instruction associated with the work using the insertion portion.

13. The object insertion system according to claim 12, **characterized in that** the second information includes information indicating an operation instruction associated with a rotation and/or an insertion/removal of the insertion portion necessary to locate a distal part of the insertion portion at a particular position in the object.

14. The object insertion system according to claim 12, **characterized in that** the insertion portion has flexibility to be at least partly curvable,
the second information includes information indicating an operation instruction associated with a curving of the insertion portion necessary to locate a distal part of the insertion portion at a particular position in the object.

15. The object insertion system according to claim 12, **characterized in that**
the fourth information includes information which is set from a perspective of a load on the object, and which indicates an upper limit value of force applied to the object by the insertion portion, and
the second information includes information indicating a relation between the upper limit value and a force actually applied to the object by the insertion portion.

16. The object insertion system according to claim 12, **characterized by** further comprising an operation portion which inserts/removes and rotates the insertion portion,
wherein the fourth information includes information which is set from a perspective of a load on the object and/or a certainty of the work and which indicates an upper limit value of an operation speed regarding an insertion/removal operation and a rotation operation of the insertion portion, and
the second information includes information indicating a relation between the upper limit value and an actual operation speed of the insertion portion.

17. The object insertion system according to claim 12, **characterized by** further comprising an operation portion which performs a curving operation of the insertion portion,
wherein the insertion portion has flexibility to be at least partly curvable,
the fourth information includes information which is set from a perspective of a load on the object and/or a certainty of the work and which indicates an upper limit value of an operation speed regarding the curving operation of the insertion portion, and
the second information includes information indicating a relation between the upper limit value and an actual operation speed of the insertion portion.

18. The object insertion system according to claim 7 or 8, **characterized in that**
the fourth information includes work target position information indicating a target position at which the insertion portion is inserted and located in the object, or a through-position through which the insertion portion reaches the target position, and
the second information calculator calculates, as the second information, information regarding a situation of adjacency, an arrival at a predetermined range, and a stray situation of the insertion portion relative to the target position or the through-position based on the work target position information and the position information.

19. The object insertion system according to claim 18, **characterized in that** the second information calculator calculates, as the second information, information indicating a change of a position of the distal part of the insertion portion relative to the target position or the through-position.

20. The object insertion system according to claim 1, **characterized in that**
the first information generator includes a bend sensor that uses an optical fiber, and
the bend sensor detects an amount of curving in one or more parts in the insertion portion.

21. The object insertion system according to claim 1, **characterized by** further comprising an operation portion which performs a curving operation of the insertion portion,
wherein the insertion portion has flexibility to be at least partly curvable,
the first information generator includes a curve sensor which detects a curving amount of the insertion portion, and an operation amount sensor which detects a curving operation amount of the operation portion, and
the second information calculator calculates a difference between an actual curving amount which is a detection result by the curve sensor and the curving operation amount detected by the operation amount sensor, and calculates, as the second information, a force applied to the object by the insertion portion based on the difference.

22. The object insertion system according to claim 1, **characterized by** further comprising:
a setting switch information input unit which inputs setting information indicating setting regarding a calculation of the second information by the second information calculator; and
a control unit which performs a control regarding a selection and/or a switch of a combination of information required for the calculation of the second information by the second information calculator based on the setting information.

23. The object insertion system according to claim 22, **characterized in that**
the setting information includes grade setting information which sets stages for at least one of contents, method, frequency, and presence of an output of the second information by the output unit, and
the control unit controls to select and/or switch regarding the calculation of the second information based on the grade setting information.

24. The object insertion system according to claim 1, **characterized by** further comprising:
a setting switch information input unit which inputs setting information indicating setting regarding a calculation of the second information by the second information calculator; and
a control unit which selects an optimum computation algorithm in the second information calculator based on the setting information to control the second information calculator.

25. The object insertion system according to claim 1, **characterized by** further comprising:
a setting switch information input unit which inputs setting information in which the second information, the first and fourth information necessary for a generation of the second information, and utilization scenes of the second information are associated; and
a control unit which controls the second information calculator to output the second information to the output unit by timing of arrival at the utilization scenes based on the setting information.

26. The object insertion system according to claim 1, **characterized by** further comprising:
a setting switch information input unit which inputs setting information in which the second information, the first and third information necessary for a generation of the second information, and utilization scenes of the second information are associated; and
a control unit which controls the second information calculator to output the second information to the output unit by timing of arrival at the utilization scenes based on the setting information.

27. The object insertion system according to claim 26, **characterized in that**
the first information includes information indicating a relative position of a distal end of the insertion portion to the object,
the third information includes information indicating a position regarding the work, and
the second information calculator calculates work target relative position information indicating a positional relation between a position of the distal end of the insertion portion and a position of the work, and determines the timing based on the work target relative position information.

28. The object insertion system according to claim 1, **characterized in that**
the output unit includes one or more kinds of output components,
the output components output the second information, and
the object insertion system further comprises a control unit which controls a calculation processing of the second information by the second information calculator and a switch processing of the output components and output methods based on the contents of the second information.

29. The object insertion system according to claim 1, **characterized in that**
the output unit includes one or more kinds of output components,
the output components output the second information,
a flag indicating urgency or importance is attached to the second information, and
the object insertion system further comprises a control unit which controls a calculation processing of the second information by the second information calculator and a switch processing of the output components and output methods in accordance with the flag.

30. The object insertion system according to claim 28 or 29, **characterized in that** the control unit controls the calculation processing and the switch processing before and after a start of the work.

31. The object insertion system according to claim 10, **characterized in that** the storage unit stores the second information calculated by the second information calculator in association with at least one of the first information, the third information, and the fifth information.

32. The object insertion system according to claim 11, **characterized by** further comprising:
a fifth information input unit to which fifth information that is new information regarding the object is input after a start of the work; and
a sixth information input unit to which sixth information that is new information regarding the work is input after the start of the work,
wherein the storage unit stores the second information calculated by the second information calculator in association with the third information and/or the fifth information as well as the fourth information and/or the sixth information.
